# EUROPEAN PATENT SPECIFICATION

(11) **EP 1 682 109 B1**
(45) Date of publication and mention of the grant of the patent: **15.10.2008**
(21) Application number: 04821452.2
(22) Date of filing: 22.10.2004
(51) Int. Cl.: A61K 31/13

(54) **THE USE OF 1-AMINOCYCLOHEXANE DERIVATIVES TO MODIFY DEPOSITION OF FIBRILLOGENIC AS PEPTIDES IN AMYLOIDOPATHIES**
VERWENDUNG VON 1-AMINOCYCLOHEXAN-DERIVATEN ZUR MODIFIZIERUNG DER ABSCHEIDUNG VON FIBRILLOGENEN AS PEPTIDEN IN AMYLOIDPATHIEN
UTILISATION DE DERIVES DE 1-AMINOCYCLOHEXANE POUR MODIFIER LE DEPOT DE PEPTIDES A_G(B) FIBRILLOGENES DANS DES AMYLOIDOPATHIES

(30) Priority: 22.10.2003 US 513700 P
(43) Date of publication of application: 26.07.2006
(73) Proprietor: Merz Pharma GmbH & Co. KGaA, 60318 Frankfurt Main (DE)
(72) Inventor: GUPTA, Sandeep, Plainsboro, NJ 08536 (US); BANARJEE, Pradeep, Hillsborough, NJ 08844 (US); LAHIRI, Debomoy, K., Indianapolis, IN 46254 (US); FARLOW, Martin, R., Indianapolis, IN 46234 (US)
(74) Representative: Marsden, John Christopher
(86) International application number: PCT/US2004/035040
(87) International publication number: WO 2005/079779

(56) References cited:
- WO-A-02/08219
- WO-A-20/04009062
- WO-A-20/04037234
- US-A- 5 061 703
- REISBERG ET AT: "Memantine in Moderate to severe Alzheimer's disease" N.ENGL.J.MED, vol. 348, 2003, - pages 1333-1341, XP009050769
- CHEN ET AL: SOC FOR NEUROSCIENCE ABTRACT VIEWER AND ITINERARY PLANNER., 2002, - 2002 XP001207129

## Description

### FIELD OF THE INVENTION

The invention relates to the use of N-methyl-D-aspartate (NMDA) receptor antagonists such as 1-aminocyclohexane derivatives to modify deposition of fibrillogenic Aβ peptides in amyloidopathies.

### BACKGROUND OF THE INVENTION

Alzheimer's disease (AD) is an increasingly prevalent form of neurodegeneration that accounts for approximately 50 % - 60 % of the overall cases of dementia among people over 65 years of age. AD is characterized clinically by progressive loss of memory, cognition, reasoning, judgement, and emotional stability that gradually leads to profound mental deterioration and ultimately death. AD is a progressive disorder with a mean duration of around 8.5 years between onset of clinical symptoms and death. AD is believed to represent the fourth most common medical cause of death and affects about 4 million people in the United States. Prevalence of AD doubles every 5 years beyond age 65 (National Institute on Aging: Prevalence and costs of Alzheimer's disease. Progress Report on Alzheimer's Disease. NIH Publication No. 99 3616, November 1998; Polvikosid et al., Neurology, 2001, 56:1690-1696). AD currently affects about 15 million people world-wide (including all races and ethnic groups) and owing to the relative increase of elderly people in the population its prevalence is likely to increase over the next two to three decades. AD is at present incurable. No treatment that effectively prevents AD or reverses its symptoms and course is currently known.

The clinical signs of AD in humans result from selective degeneration of neurons in brain regions associated with higher mental functions such as memory, cognitive performance and personality (Francis et al., 1999, J. Neurol. Neurosurg. Psychiatry, 66:137-147). Dysfunction and death of these neurons leads to reduced numbers of synaptic markers in their target fields; the disruption of synaptic communication is manifested by mental impairments and, finally, severe dementia.

The brains of individuals with AD exhibit characteristic lesions termed senile (or amyloid) plaques, amyloid angiopathy (amyloid deposits in blood vessels) and neurofibrillary tangles. Smaller numbers of these lesions in a more restricted anatomical distribution are also found in the brains of most aged humans who do not have clinical AD. Amyloid plaques and amyloid angiopathy also characterize the brains of individuals with Trisomy 21 (Down's Syndrome) and Hereditary Cerebral Hemorrhage with Amyloidosis of the Dutch-Type (HCHWA-D).

Two types of protein aggregates found in the brain are pathological hallmark of AD: intracellular neurofibrillary tangles and extracellular amyloid plaques (for a recent review *see* Wong et al., Nature Neurosci., 2002, 5: 633-639). Both tangles and plaques are preferentially localized to the cortex, hippocampus and amygdala. Neurofibrillary tangles are inclusions located within cell bodies and proximal dendrites, and within filamentous swellings in distal axons and synaptic terminals. Hyperphosphorylated isoforms of the microtubule-associated protein *tau*, which assemble into poorly soluble paired helical filaments, are a central feature of these neurofibrillary tangles (Goedert et al., Curr. Opin. Neurobiol., 1998, 8: 619-632).

The extracellular plaques result from elevated levels of an approximately 4.2 kilodalton (kD) protein of about 39-43 amino acids designated the β-amyloid peptide (Aβ) or sometimes βAP, AβP or β/A4 (*see*, *e.g.*, Glenner and Wong, Biochem. Biophys. Res. Commun., 120:885-890, 1984; U.S. Patent No. 4,666,829). Molecular biological and protein chemical analyses have shown that Aβ is a small fragment of a much larger precursor protein, referred to as the β-amyloid precursor protein (APP) (*see*, *e.g.*, Lahiri et al., Drug Dev. Res., 56:267-281, 2002; Selkoe, Physiol. Rev., 81:741-766, 2001). APP is a type I transmembrane protein normally expressed in many different cell types, but particularly abundant in neurons. Aβ monomers form oligomers and multimers, which assemble into protofilaments and then fibrils. Eventually, Aβ fibrils are deposited as the amyloid cores of neuritic or senile plaques (amyloidosis), which are complex structures also containing dystrophic neurites, astrocytes and microglia. Amyloid peptides are generated via cleavage of APP by three different proteases, termed α-, β- and γ-secretases. β-secretase, cleaves APP on the amino side of Aβ producing a large secreted derivative, sAPPβ, and an Aβ-bearing membrane-associated C-terminal derivative, CTFβ, which is subsequently cleaved by the second activity, γ-secretase, to release Aβ. Alternatively, a third activity, α-secretase, cleaves APP within Aβ to the secreted derivative sAPPα and membrane-associated CTFα. The predominant secreted APP derivative is sAPPα in most cell types. Most of the secreted Aβ is 40 residues long (Aβ₄₀) although a small percentage is 42 residues in length (Aβ₄₂). However, the longer Aβ₄₂ aggregates more readily and is therefore considered to be the pathologically important form (for a recent review see Sambamurti et al., Neuromolecular Med., 1:1-31, 2002). The APP-processing events just summarized are entirely normal and occur to varying degrees in virtually all neural and non-neural cells throughout the body. Certain genetic defects that cause autosomal dominant AD, such as mutations in APP or the presenilin (PS) genes PS1 and PS2, augment the amyloidogenic pathway of APP processing in all cells in a way that favors production of the highly self-aggregating Aβ₄₂ variant over the slightly shorter and less hydrophobic Aβ₄₀ form. Aβ₄₂ normally comprises only about 5-10% of total secreted Aβ peptides, but this fraction rises to about 15-40% when either APP or PS is mutant (Selkoe, J. Clin. Invest., 110:1375-81, 2002).

If Aβ peptides, particularly Aβ₄₂, are overproduced or insufficiently cleared, they become prone to aggregation into stable oligomers and larger polymers, apparently culminating in mature amyloid fibrils. Oligomeric intermediates of Aβ, rather than mature amyloid fibrils, may turn out to be the principal form through which the peptide exerts its il1 effects. Aβ oligomers may exert complex effects on surrounding neurons, microglia, and astrocytes, the cumulative effect of which is to subtly alter synaptic function, and thus information storage and retrieval. As supported by recent studies of mice bearing both human Aβ and human tau, the tau-containing dystrophic neurites and neurofibrillary tangles that develop in "thinking" parts of the brain in AD are likely to be a consequence of Aβ build-up (Selkoe, 2002, *supra*, and references therein).

One of the current therapeutic strategies in AD is a reduction in the levels of the toxic Aβ. These include decreasing or preventing the release of Aβ peptide by either increasing α-secretase or decreasing the β- or γ-secretase activity or production (*e.g.*, by using small-molecule inhibitors). Other strategies include decreasing Aβ peptide aggregation, increasing Aβ peptide clearance, reducing Aβ peptide production or decreasing the cellular effects of Aβ peptide aggregation and deposition. (*see*, *e.g.*, Sabbagh et al., Alzheimer's Disease Rev., 3:1-19, 1997; U.S. Patent No. 6,080,778). These approaches include active or passive "Aβ vaccination", which derives from mouse studies in which the repetitive parenteral administration of synthetic Aβ peptide was found to induce an antibody response that lowered cerebral Aβ levels (Schenk et al., Nature, 400:173-177, 1999). Also, the inventors and co-workers have shown that tacrine, an inhibitor of the cholinergic catabolic enzyme acetylcholinesterase (AChEI), is able to reduce the release of the secreted form of APP, sAPPα, and total Aβ, Aβ₄₀ and Aβ₄₂ in human neuroblastoma cells in the absence of any detectable cellular damage or toxicity (Lahiri et al., Mol. Brain Res. 1998, 62: 131-140).

The latter results can be linked to the fact that AD is associated with a profound loss of cholinergic neurons within the nucleus basalis of Meynert (Perry et al., Br. Med. J., 1978, 2:1456-1459; Geula and Mesulam, Cholinergic systems and related neuropathological predilection patterns in Alzheimer disease; In: Alzheimer's Disease; Terry et al. eds., Raven Press, New York, 1994, pp. 263-291).

The excessive or pathological activation of glutamate receptors, particularly those that are selectively activated by N-methyl-D-aspartate (NMDA), has also been implicated in the processes that underlie the degeneration of cholinergic cells in the brains of AD patients (Greenamyre et al., Neurobiol. Aging, 1989, 10:593-602; Francis et al., J. Neurochem., 1993, 60:263-291; Li et al., J. Neuropathol. Exp. Neurol., 1997, 56:901-911; Wu and Rowan, Neuroreport,1995, 6:2409-2413). There is also evidence that Aβ enhances glutamate toxicity and augments NMDA receptor-mediated neurotoxicity. Indeed, NMDA receptors have been implicated in the signalling cascades affecting or affected by APP processing. Thus, in cultured hippocampal neurons, sAPPα, has been shown to selectively suppress NMDA-mediated currents (Furukawa and Mattson, Neuroscience, 1998, 83: 429-438).

Based on their earlier data showing the ability of AChEIs to reduce the release of the Aβ in human neuroblastoma cells, the present inventors have hypothesized that NMDA receptor antagonists can be also useful to reduce the levels of the toxic Aβ and therefore treat and/or prevent AD as well as other amyloidopathies.

Functional inhibition of NMDA receptors can be achieved through actions at different recognition sites within the NMDA receptor complex, such as: the primary transmitter site (competitive), the phencyclidine site located inside the cation channel (uncompetitive), the polyamine modulatory site and the strychnine-insensitive, co-agonistic glycine site (glycine B) (Parsons *et al.*, 1999, *supra*). As NMDA receptors also play a crucial physiological role in various forms of synaptic plasticity such as those involved in learning and memory (*see*, *e.g.*, Collingridge and Singer, Trends Pharmacol. Sci., 1990, 11:290-296), neuroprotective agents possessing high affinity for the NMDA receptors are likely to impair normal synaptic transmission and thereby cause numerous side effects. Indeed, many NMDA receptor antagonists identified to date produce highly undesirable side effects at doses within their putative therapeutic range. Thus, clinical trials showed diminished overall therapeutic utility (despite efficacy) due to numerous side effects for such NMDA receptor antagonists as Dizocilpine ((+)MK-801; (+)-5-methyl- 10, 11 -dihydro-5H-dibenzocyclohepten-5,10-imine maleate), Cerestat (CNS-1102), Licostinel (ACEA 1021), Selfotel (CGS-19755), and D-CPP-ene (Leppik , Epilepsia, 1998, 39 (Suppi 5):2-6; Sveinbjornsdottir et al., Epilepsia, 1993, 34:493-521; SCRIP 2229/30, 1997, p. 21). The challenge in the field has therefore been to develop NMDA receptor antagonists that prevent the pathological activation of NMDA receptors but allow their physiological activity.

Memantine (1-amino-3,5-dimethyl adamantane) is an analog of 1-aminocyclohexane (disclosed, *e.g.*, in U.S. Patents No. 4,122,193; 4,273,774; 5,061,703). Neramexane (1-amino-1,3,3,5,5-pentamethylcyclohexane) is also a derivative of 1-aminocyclohexane (disclosed, *e.g.*, in U.S. Patent No. 6,034,134). Memantine, related adamantane derivatives, neramexane as well as some other 1-aminoalkyl-cyclohexanes are systemically-active uncompetitive NMDA receptor antagonists having low to moderate affinity for the receptor. They exhibit strong voltage-dependent receptor blocking characteristics and fast receptor blocking/unblocking kinetics (Parsons *et al.*, 1999, *supra*; Görtelmeyer et al., Arzneim-Forsch/Drug Res., 1992, 42:904-913; Winblad et al., Int. J. Geriat. Psychiatry, 1999, 14:135-146; Rogawski, Amino Acids, 2000, 19: 133-49; Danysz et al., Curr. Pharm. Des., 2002, 8:835-43; Jirgensons et al., Eur. J. Med. Chem., 2000, 35: 555-565). These compounds dissociate from the NMDA receptor channels much more rapidly than the high affinity NMDA receptor antagonists such as (+)MK-801 and attenuate disruption of neuronal plasticity produced by tonic overstimulation of NMDA receptors. Due to their relatively low affinity for the receptor and strong voltage-dependent fast receptor unblocking kinetics, these compounds are essentially devoid of the side effects of other NMDA receptor antagonists at therapeutic doses (Kornhuber et al., Eur. J. Pharmacol., 1991, 206:297-311). Indeed, memantine has been applied clinically for over 15 years showing good tolerability with the number of treated patients exceeding 200,000 (Parsons *et al.*, 1999, *supra*).

Memantine, neramexane as well as other 1-aminoalkylcyclohexanes have been suggested to be useful in alleviation of various progressive neurodegenerative disorders such as dementia in AD, Parkinson's disease, and spasticity (*see*, *e.g.*, U. S. Patents No. 5,061,703; 5,614,560, and 6,034,134; Parsons *et al.*, 1999, *supra*; Möbius, ADAD, 1999,13:S172-178; Danysz et al., Neurotox. Res., 2000, 2:85-97; Winblad and Poritis, Int. J. Geriatr. Psychiatry, 1999, 14:135-146; Görtelmeyer *et al.*, 1992, *supra*; Danysz et al., Curr. Pharm. Des., 2002, 8:835-843; Jirgensons et al., Eur. J. Med. Chem., 2000, 35: 555-565). These diseases are thought to be causally associated (and in any event are closely correlated) with disturbances of glutamatergic transmission, *i.e.*, the excessive influx of calcium through NMDA receptor channels, leading to the destruction of brain cells in specific brain areas (Choi, J. Neurobiol., 23: 1261-1276, 1992; Rothman and Olney, Trends Neurosci., 10: 299, 1987; Kemp et al., Trends Pharmacol. Sci., 8: 414,1987). Chronic treatment of aged rats with memantine has been shown to enhance the formation of hippocampal long-term potentiation, increase the durability of synaptic plasticity, improve spatial memory abilities, and reverse the memory impairment produced by NMDA receptor agonists (Barnes et al., Eur. J. Neurosci.,1996; 8:65-571; Zajaczkowski et al., Neuropharm., 1997,36:961-971).

Despite abundant data on their clinical effects, the ability of NMDA receptor antagonists to affect directly the deposition of fibrillogenic Aβ peptides has not been suggested. Also, there is clearly a need in the art for a more effective treatment of mammals suffering from amyloidopathies. The present inventors have satisfied this need by conceiving and demonstrating for the first time that NMDA receptor antagonists such as 1 -aminocyclohexane derivatives (*e.g.*, memantine or neramexane) are able to decrease the levels of secreted sAPP and Aβ₄₀ (and possibly Aβ₄₂) and therefore modify deposition of fibrillogenic Aβ peptides. These findings support the idea that, in addition to providing symptomatic relief in patients, NMDA receptor antagonists such as 1-aminocyclohexane derivatives may directly modify the underlying pathology of amyloid deposition.

### BRIEF DESCRIPTION OF THE DRAWINGS

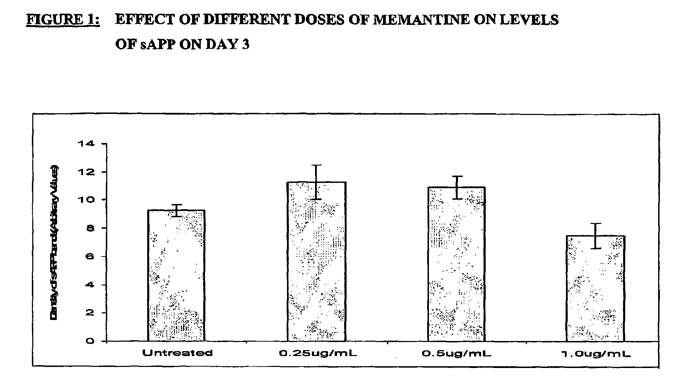

**Figure 1** shows the effect of different doses of memantine (0 µg/ml, 0.25 µg/ml, 0.5 µg/ml, and 1 µg/ml) on the levels of sAPP in the conditioned media of human neuroblastoma cells SK-N-SH on day 3 (as determined by Western blot analysis using specific mAb against either total APP (mAb22C11) or sAPPα (mAb6E10)).

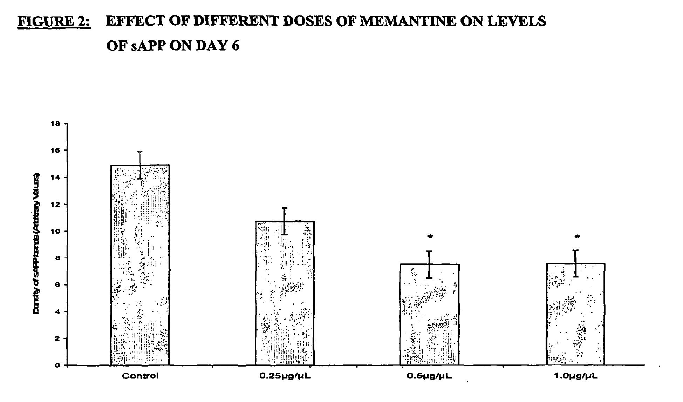

**Figure 2** shows the effect of different doses of memantine (0 µg/ml, 0.25 µg/ml, 0.5 µg/ml, and 1 µg/ml) on the levels of sAPP in the conditioned media of human neuroblastoma cells SK-N-SH on day 6 (as determined by Western blot analysis using specific mAb against either total APP (mAb22C11) or sAPPα (mAb6E10)).

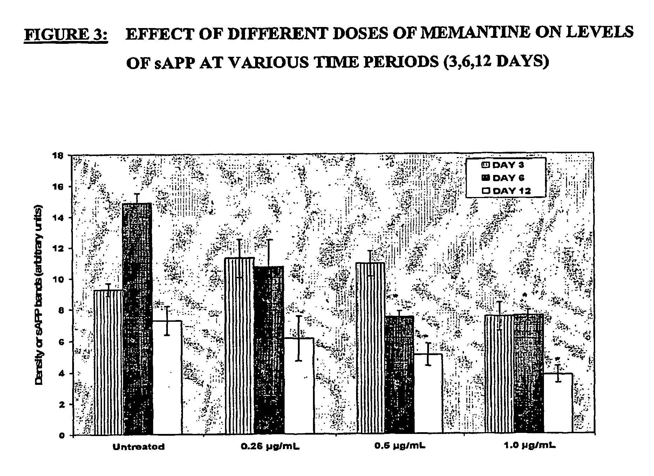

**Figure 3** shows the effect of different doses of memantine (0 µg/ml, 0.25 µg/ml, 0.5 µg/ml, and 1 µg/ml) on the levels of sAPP in the conditioned media of human neuroblastoma cells SK-N-SH at various time periods (3,6,12 days) (as determined by Western blot analysis using specific mAb against either total APP (mAb22C11) or sAPPα (mAb6E10)).

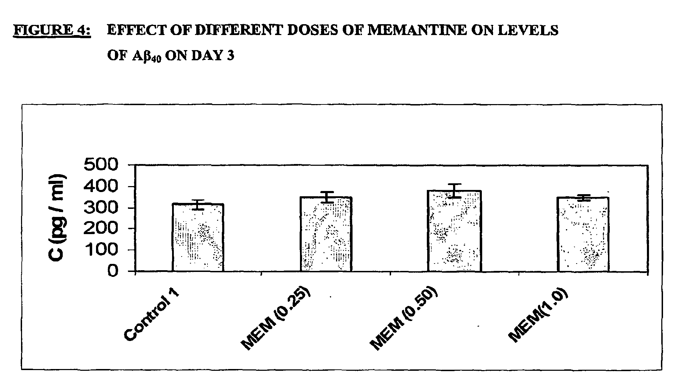

**Figure 4** shows the effect of different doses of memantine (0 µg/ml, 0.25 µg/ml, 0.5 µg/ml, and 1 µg/ml) on the levels of Aβ₄₀ in the conditioned media of human neuroblastoma cells SK-N-SH on day 3 (as determined by ELISA using anti-human Aβ (35-40) rabbit IgG as a capture Ab and HRP conjugated anti-human Aβ (11-28) rabbit IgG Fab as a detection Ab).

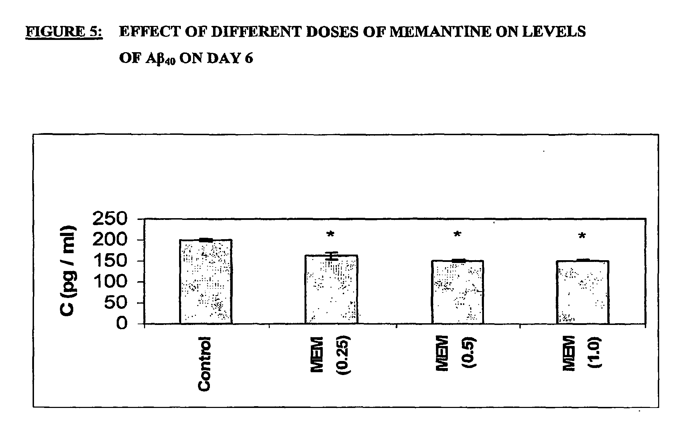

**Figure 5** shows the effect of different doses of memantine (0 µg/ml, 0.25 µg/ml, 0.5 µg/ml, and 1 µg/ml) on the levels of Aβ₄₀ in the conditioned media of human neuroblastoma cells SK-N-SH on day 6 (as determined by ELISA using anti-human Aβ (35-40) rabbit IgG as a capture Ab and HRP conjugated anti-human Aβ (11-28) rabbit IgG Fab as a detection Ab).

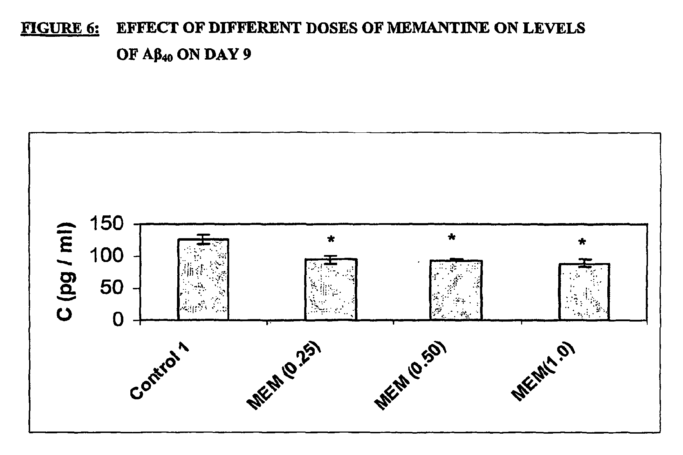

**Figure 6** shows the effect of different doses of memantine (0 µg/ml, 0.25 µg/ml, 0.5 µg/ml, and 1 µg/ml) on the levels of Aβ₄₀ in the conditioned media of human neuroblastoma cells SK-N-SH on day 9 (as determined by ELISA using anti-human Aβ (35-40) rabbit IgG as a capture Ab and HRP conjugated anti-human Aβ (11-28) rabbit IgG Fab as a detection Ab).

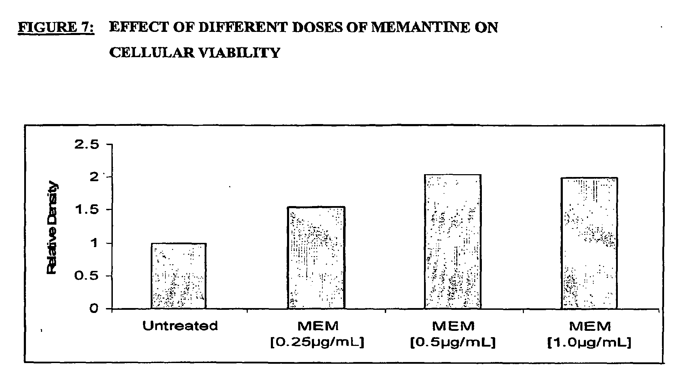

**Figure 7** shows the effect of different doses of memantine (0 µg/ml, 0.25 µg/ml, 0.5 µg/ml, and 1 µg/ml) on cellular viability of human neuroblastoma cells SK-N-SH as measured by MTT (the tetrazolium dye 3-[4,5-dimethylthiazol-2-yl]-2,5-diphenyltetrazolium bromide) assay.

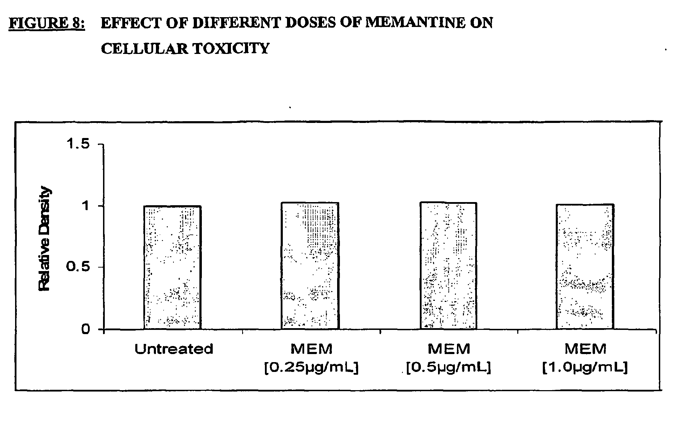

**Figure 8** shows the effect of different doses of memantine (0 µg/ml, 0.25 µg/ml, 0.5 µg/ml, and 1 µg/ml) on cellular toxicity of human neuroblastoma cells SK-N-SH as measured by LDH (lactate dehydrogenase) assay.

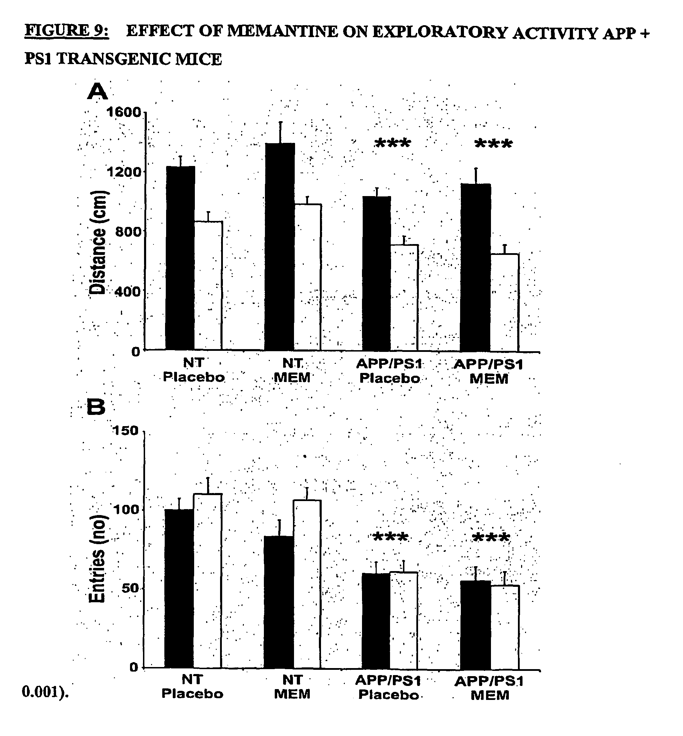

**Figure 9** shows measures of exploratory activity during a 10-min recording in a closed transparent cage. (A) Horizontal activity (distance traveled), (B) vertical activity (number of rearings). The filled and the open columns denote mean ± SEM on Day 1 and Day 3, respectively. ***APP/PS1 mice differed significantly from the NT littermates in the overall ANOVA (p< 0.001).

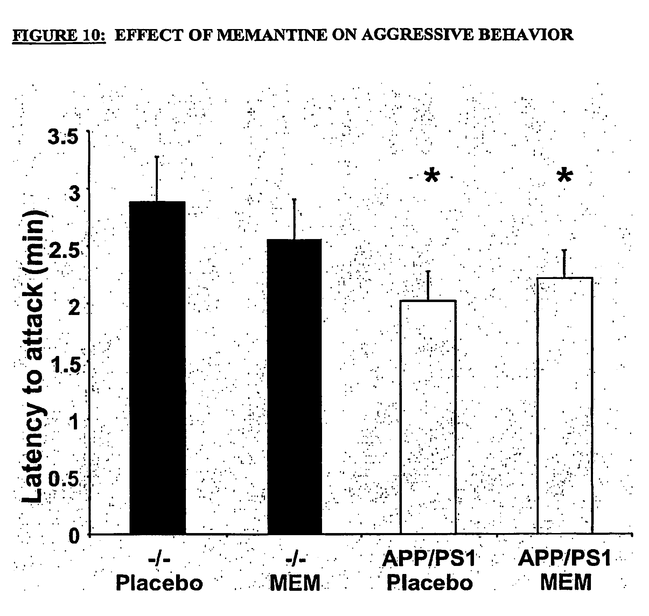

**Figure 10** shows an isolation-induced aggression test. The columns denote mean + SEM latency of the resident mouse to attack the intruder mouse (filled columns for NT mice and open columns for APP/PS1 mice). *APP/PS1 mice differed significantly from the NT littermates in the overall ANOVA (p<0.05).

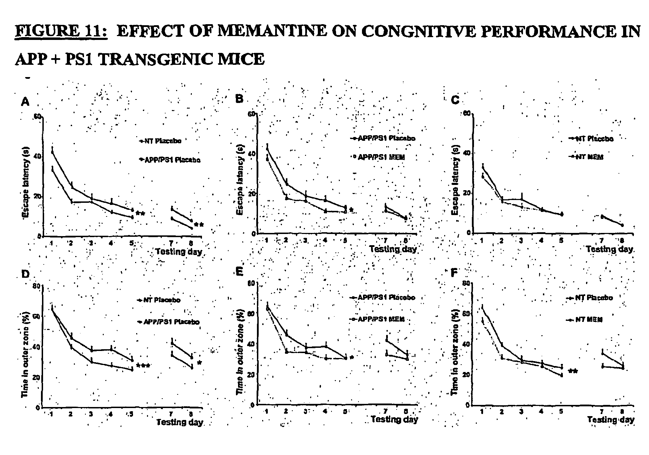

**Figure 11** shows the Morris water maze test for memantine and placebo-treated NT and APP/PS1 mice. The mean escape latency (A, B, C) and mean % time in the outer zone of the pool (D, E, F) are given for different test days. Days 1-5: hidden platform test; days 7-8, visible platform test. The asterisks denote differences between the given two groups over all testing days (five for hidden platform, two for visible platform): *p < 0.05, **p < 0.01, ***p < 0.001 (ANOVA for repeated measures).

### SUMMARY OF THE INVENTION

The instant invention provides for the use of of a 1-aminocyclohexane derivative in the manufacture of a medicament for decreasing the level of at least one amyloid peptide produced by a mammalian cell that expresses amyloid precursor protein, such as sAPPα, Aβ₄₀ or Aβ₄₂, more particularly for treating, preventing, arresting, delaying the onset of and/or reducing the risk of developing an amyloidopathy selected from diffuse Lewy body disease, Creutzfeldt-Jakob disease, familial amyloidosis of Finnish type, familial amyloidotic polyneuropathy and hereditary cerebral hemorrhage with amyloidosis of the Dutch type in a mammal. Preferably, the 1-aminocyclohexane derivative is represented by the general formula (I): 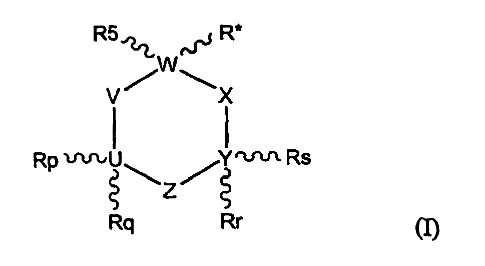 wherein:
- R* is -(A)ₙ-(CR¹R²)ₘ-NR³R⁴,
   n+m=0, 1, or 2,
   A is selected from the group consisting of linear or branched lower alkyl (C₁-C₆),linear or branched lower alkenyl (C₂-C₆), and linear or branched lower alkynyl (C₂-C₆),
   R¹ and R² are independently selected from the group consisting of hydrogen, linear or branched lower alkyl (C₁-C₆), linear or branched lower alkenyl (C₂-C₆), linear or branched lower alkynyl (C₂-C₆) aryl, substituted aryl and arylalkyl,
   R³ and R⁴ are independently selected from the group consisting of hydrogen, linear or branched lower alkyl (C₁-C₆), linear or branched lower alkenyl (C₂-C₆), and linear or branched lower alkynyl (C₂-C₆), or together form alkylene (C₂-C₁₀) or alkenylene (C₂-C₁₀) or together with the N form a 3-7-membered azacycloalkane or azacycloalkene, including substituted (alkyl (C₁-C₆), alkenyl (C₂-C₆)) 3-7-membered azacycloalkane or azacycloalkene; or independently R³ or
   R⁴ may join with R^{p}, R^{q}, R^{r}, or R^{s} to form an alkylene chain -CH(R⁶)-(CH₂)ₜ-, wherein t= 0 or 1 and the left side of the alkylene chain is attached to U or Y and the right side of the alkylene chain is attached to N and R⁶ is selected from the group consisting of hydrogen, linear or branched lower alkyl (C₁-C₆), linear or branched lower alkenyl (C₂-C₆), linear or branched lower alkynyl (C₂-C₆), aryl, substituted aryl and arylalkyl; or independently R³ or R⁴ may join with R⁵ to form an alkylene chain represented by the formula -CH₂-CH₂-CH₂-(CH₂)ₜ-, or an alkenylene chain represented by the formulae -CH=CH-CH₂-(CH₂)ₜ-, -CH=C=CH-(CH₂)ₜ- or -CH₂-CH=CH-(CH₂)ₜ-, wherein t= 0 or 1, and the left side of the alkylene or alkenylene chain is attached to W and the right side of the alkylene ring is attached to N;
- R⁵ is independently selected from the group consisting of hydrogen, linear or branched lower alkyl (C₁-C₆), linear or branched lower alkenyl (C₂-C₆), and linear or branched lower alkynyl (C₂-C₆), or R⁵ combines with the carbon to which it is attached and the next adjacent ring carbon to form a double bond,
- R^{p}, R^{q}, R^{r}, and R^{s}, are independently selected from the group consisting of hydrogen, linear or branched lower alkyl (C₁-C₆), linear or branched lower alkenyl (C₂-C₆), linear or branched lower alkynyl (C₂-C₆), cycloalkyl (C₃-C₆) and aryl, substituted aryl and arylaklyl or R^{p}, R^{q}, R^{r}, and R^{s} independently may form a double bond with U or with Y or to which it is attached, or R^{p}, R^{q}, R^{r}, and R^{s} may combine together to represent a lower alkylene -(CH₂)ₓ- or a lower alkenylene bridge wherein x is 2-5, inclusive, which alkylene bridge may, in turn, combine with R⁵ to form an additional lower alkylene - (CH₂)_{y}- or a lower alkenylene bridge, wherein y is 1-3, inclusive,
- the symbols U, V, W, X, Y, Z represent carbon atoms,
and include optical isomers, diastereomers, polymorphs, enantiomers, hydrates, pharmaceutically acceptable salts, and mixtures of compounds within formula (I).

Most preferred NMDA receptor anatgonists for use in the present invention are memantine and neramexane. Also preferably, the cell is of a neuronal origin.

In conjunction with the first method, the invention provides a novel method for modifying potential deposition of fibrillogenic β-amyloid (Aβ) peptides in a mammal comprising administering to said mammal an 1-aminocyclohexane derivative in amounts effective for this purpose. Preferably, the 1-aminocyclohexane derivative is administered in amounts, which are in the range 0.1-150 µM, more preferably in the range 1-25 µM, and most preferably in the range 1-4 µM. In a specific embodiment, the mammal is a mouse or a human.

The invention further provides a method for potentially treating, preventing, arresting, delaying the onset of and/or reducing the risk of developing an amyloidopathy other than an amyloidopathy associated with Alzheimer's disease (AD) in a mammal comprising administering to said mammal an 1-aminocyclohexane derivative in amounts effective for this purpose. In a specific embodiment, the amyloidopathy includes but is not limited to Down's Syndrome, diffuse Lewis body disease, progressive supranuclear palsy, , Creutzfeldt-Jakob disease, familial amyloidosis of Finnish type, familial amyloidotic polyneuropathy, hereditary cerebral hemorrhage with amyloidosis of the Dutch type, and Gerstmann-Straussler Scheinker syndrome. According to a specific embodiment, the 1-aminocyclohexane derivative is administered in therapeutically effective dosages, which are in the range 1-100 mg/day, most preferably, in the range 5-60 mg/day and especially at 10-40 mg/day.

Accordingly, one object of the instant invention is to administer a 1-aminocyclohexane derivative to human subjects who either do not yet show clinical signs of an amyloidopathy, but who are at risk of developing elevated levels of potentially toxic and fibrillogenic Aβ, or to individuals who may already show signs of cognitive impairment or may be at risk of such impairment due to having elevated levels of Art By providing the 1-aminocyclohexane derivative, the invention provides compositions and methods for possibly reducing the risk of developing an amyloidopathy or delaying the onset of amyloidopathy in such individuals. In addition, as disclosed herein, such therapy may halt or reduce the rate of further cognitive decline and, over a period of time, reverse cognitive decline, as measured by at least one marker or method. Examples of such symptoms or markers are patients' ADL, SIB, MMSE, CIBIC or ADAScog scores.

In a specific embodiment, the invention relates to a method for treating a mammal having an amyloidopathy other than an amyloidopathy associated with Alzheimer's disease which comprises lowering the amount of Aβ peptides in the brain, cerebrospinal fluid, or plasma of the mammal by administering to the mammal a composition comprising a therapeutically effective amount of a 1-aminocyclohexane derivative. Lowering the amount of Aβ peptides in the brain may comprise affecting APP processing.

In another embodiment, the invention relates to a method for treating a mammal having an amyloidopathy which comprises increasing the clearance of Aβ peptides in the brain, cerebrospinal fluid, or plasma of the mammal by administering to the mammal a composition comprising a therapeutically effective amount of a 1-aminocyclohexane derivative. In a preferred embodiment, the clearance of Aβ peptides in the brain of the mammal is increased.

In yet another embodiment, the invention relates to a method for treating a mammal having an amyloidopathy other than an amyloidopathy associated with Alzheimer's disease comprising preventing or reducing Aβ peptide aggregation or plaque formation in the brain of the mammal by administering to the mammal a composition comprising a therapeutically effective amount of a 1-aminocyclohexane derivative.

In another embodiment, the invention relates to a method for the treatment of a mammal exhibiting the objective symptoms of an amyloidopathy other than an amyloidopathy associated with Alzheimer's disease by decreasing the formation of Aβ peptides, increasing the clearance of Aβ peptides, regulating the processing of APP, or reducing plaque maturation in the mammal by administering to the mammal a composition comprising a therapeutically effective amount of a 1-aminocyclohexane derivative.

In certain embodiments, the detected Aβ level is decreased by about 10-70% or more.

According to a separate embodiment, the 1-aminocyclohexane derivative is administered in combination (simultaneously or sequentially) with another 1-aminocyclohexane derivative, an acetylcholinesterase inhibitor (AChEI), a secretase modifier (*e.g.*, β- and/or γ-secretase inhibitor, β-sheet breaker, or α-secretase enhancer), or a combination thereof. The acetylcholinesterase inhibitors (ACHEI) useful for the method of the invention include but are not limited to galantamine, tacrine, donepezil, physostigmine and rivastigmine.

In other related embodiments, the present invention provides for a method of managing the 1-aminocyclohexane derivative treatment of a patient with an amyloidopathy. Preferably, the present invention provides a method for monitoring the effect of a therapeutic treatment on a subject who has undergone therapeutic treatment with a 1-aminocyclohexane derivative. This method comprises measuring at suitable time intervals the amount of Aβ concentration in a body fluid. Any change or absence of change in the amount of the Aβ can be identified and correlated with the effect of the therapeutic treatment on the subject. In certain preferred embodiments the present invention involves detecting a change or no change in Aβ levels, in the 1-aminocyclohexane derivative therapy and adjusting the therapy accordingly. The measured amount of Aβ can be compared to a baseline level. Preferably, this baseline level of Aβ concentration is the level present in the subject prior to 1-aminocyclohexane derivative therapy. In certain embodiments, the baseline level is the level measured in a patient on existing 1-aminocyclohexane derivative therapy.

In conjunction with the methods of the invention, provided herein are pharmaceutical compositions and administration dosages comprising a therapeutically effective amount of at least one 1-aminocyclohexane derivative (*e.g.*, memantine or neramexane) and, optionally, a pharmaceutically acceptable carrier or excipient. Also provided are soluble β-amyloid peptide detection means (*e.g.*, anti-Aβ antibodies), as well as detection assays and kits comprising said detection means for using in screening and therapeutic evaluations of 1-aminocyclohexane derivative treatment methods of the invention.

### DETAILED DESCRIPTION OF THE INVENTION

### Methods of the Invention

The present inventors have discovered that NMDA receptor antagonists such as 1-aminocyclohexane derivatives (*e.g.*, memantine or neramexane) lower the levels of secreted Aβ peptides (*e.g.*, by preventing or reducing Aβ formation, increasing Aβ clearance, preventing or reducing Aβ aggregation, or combination thereof).

Accordingly, the present invention provides a novel method for decreasing the level of at least one amyloid peptide produced by a mammalian cell, said method comprising administering to said cell an 1-aminocyclohexane derivative. In a specific embodiment, said amyloid peptide is sAPPα or Aβ (*e.g.*, Aβ₄₀ or Aβ₄₂). Preferably, the 1-aminocyclohexane derivative is memantine or neramexane. Also preferably, the cell is a neural cell.

In conjunction with the first method, the invention provides a novel method for potentially reducing deposition of fibrillogenic β-amyloid (Aβ) peptides in a mammal comprising administering to said mammal an 1-aminocyclohexane derivative in amounts effective for this purpose. Preferably, the 1-aminocyclohexane derivative is administered in amounts, which are in the range 0.1-150 µM, more preferably in the range 1-25 µM, and most preferably in the range 1-4 µM. In a specific embodiment, the mammal is a mouse or a human.

The invention further provides a method for possibly treating, preventing, arresting, delaying the onset of and/or reducing the risk of developing an amyloidopathy in a mammal comprising administering to said mammal an 1-aminocyclohexane derivative in amounts effective for this purpose. In a specific embodiment, the amyloidopathy includes but is not limited to Alzheimer's disease (AD), Parkinson's disease, , Down's Syndrome, diffuse Lewis body disease, progressive supranuclear palsy, Creutzfeldt-Jakob disease, familial amyloidosis of Finnish type, familial amyloidotic polyneuropathy, Hereditary cerebral hemorrhage with amyloidosis of the Dutch type, and Gerstmann-Straussler Scheinker syndrome. Preferably, the mammal is human. According to a specific embodiment, the 1-aminocyclohexane derivative is administered in therapeutically effective dosages, which are in the range 1-100 mg/day, most preferably, in the range 5-60 mg/day and especially at 10-40 mg/day.

Accordingly, one object of the instant invention is to administer a 1-aminocyclohexane derivative to human subjects who either do not yet show clinical signs of an amyloidopathy, but who are at risk of developing elevated levels of fibrillogenic Aβ, or to individuals who may already show signs of cognitive impairment or may be at risk of such impairment due to having elevated levels of Aβ. By providing the the 1-aminocyclohexane derivative, the invention provides compositions and methods for reducing the risk of developing an amyloidopathy or delaying the onset of amyloidopathy in such individuals. In addition, as disclosed herein, such therapy may halt or reduce the rate of further cognitive decline and, over a period of time, reverse cognitive decline, as measured by at least one marker or method. Examples of such symptoms or markers are patients' ADL, SIB, MMSE, CIBIC or ADAScog scores.

In a specific embodiment, the invention relates to a method for treating a mammal having an amyloidopathy which comprises lowering levels of Aβ peptides in the brain, cerebrospinal fluid, or plasma of the mammal by administering to the mammal a composition comprising a therapeutically effective amount of a 1-aminocyclohexane derivative. Lowering the amount of Aβ peptides in the brain may comprise affecting APP processing. In a preferred embodiment, the amount of Aβ peptides is lowered in the brain of the mammal.

In another embodiment, the invention relates to a method for treating a mammal having an amyloidopathy which comprises increasing the clearance of Aβ peptides in the brain, cerebrospinal fluid, or plasma of the mammal by administering to the mammal a composition comprising a therapeutically effective amount of a 1-aminocyclohexane derivative. In a preferred embodiment, the clearance of Aβ peptides in the brain of the mammal is increased.

In yet another embodiment, the invention relates to a method for treating a mammal having an amyloidopathy comprising preventing or reducing Aβ peptide aggregation or plaque formation in the brain of the mammal by administering to the mammal a composition comprising a therapeutically effective amount of a 1-aminocyclohexane derivative.

In another embodiment, the invention relates to a method for a potential treatment of a mammal exhibiting the objective symptoms of an amyloidopathy by decreasing the formation of Aβ peptides, increasing the clearance of Aβ peptides, regulating the processing of APP, or reducing plaque formation in the mammal by administering to the mammal a composition comprising a therapeutically effective amount of a 1-aminocyclohexane derivative.

In certain embodiments, the detected Aβ level is decreased by about 10-70% or more.

According to a separate embodiment, the 1-aminocyclohexane derivative is administered in combination (simultaneously or sequentially) with another 1-aminocyclohexane derivative, an acetylcholinesterase inhibitor (AChEI), a secretase modifier (*e.g.*, β- and/or γ-secretase inhibitor, β-sheet breaker, or α-secretase enhancer), or a combination thereof. The acetylcholinesterase inhibitors (AChEI) useful for the method of the invention include but are not limited to galantamine, tacrine, donepezil, and rivastigmine.

In other related embodiments, the present invention provides for a method of managing the 1-aminocyclohexane derivative treatment of a patient with an amyloidopathy. Preferably, the present invention provides a method for monitoring the effect of a therapeutic treatment on a subject who has undergone therapeutic treatment with a 1-aminocyclohexane derivative. This method comprises measuring at suitable time intervals the levels of secreted Aβ in a body fluid. Any change or absence of change in the amount of the Aβ can be identified and correlated with the effect of the therapeutic treatment on the subject. In certain preferred embodiments the present invention involves detecting a change or no change in Aβ levels, in the 1-aminocyclohexane derivative therapy and adjusting the therapy accordingly. The measured amount of Aβ can be compared to a baseline level. Preferably, this baseline level (concentration) of Aβ is the level present in the subject prior to 1-aminocyclohexane derivative therapy. In certain embodiments, the baseline level is the level measured in a patient on existing 1-aminocyclohexane derivative therapy.

In certain embodiments, the invention comprises comparing a detected level of Aβ in a body fluid of a mammal with at least one previously detected level of Aβ in order to determine the efficacy of the 1-aminocyclohexane derivative administration. The detected level can also be compared to an accepted value known in the art which is accepted as normal or indicative of the disease state. In further embodiments, the invention comprises adjusting the repeated dosing of 1-aminocyclohexane derivative based on said comparison.

Any procedures known in the art for the measurement of amyloid peptide levels can be used in the practice of the instant invention. Such procedures include but are not limited to competitive and non-competitive assay systems using techniques such as radioimmunoassays, ELISA (enzyme linked immunosorbent assay), "sandwich" immunoassays, precipitin reactions, gel diffusion precipitin reactions, immunodiffusion assays, agglutination assays, complement-fixation assays, immunoradiometric assays, fluorescent immunoassays, western blots, protein A immunoassays, and immunoelectrophoresis assays, combinations thereof and the like. Generally speaking, the method for quantitative measurement of amyloid peptide involves capture of the amyloid peptide with a first capture-antibody, washing away all unbound components, and detecting the remaining complex with a second detection-antibody. Preferably, the immunoassay designs are based on numerous "capture and detection-antibody" combinations, and may involve combinations of antibodies, provided that each antibody reacts with separate epitopes.

In a specific embodiment, the method of the invention comprises using Aβ peptide antibodies to capture and detect the presence of Aβ in the body fluid. Accordingly, in a particular aspect, the present invention provides specific binding assays which are useful for the measurement of Aβ concentrations in fluid samples and which may be employed in both the screening and diagnostic methods of the invention. The specific binding assay of the present invention is capable of detecting soluble Aβ at the very low concentrations which are characteristic of the patient fluids and conditioned culture media, typically being capable of measuring threshold concentrations in the range from about 1 ng/ml to 10 ng/ml, or lower. Specific binding assays according to the present invention employ at least one binding substance specific for an epitope or determinant site on the Aβ molecule, which site is generally not found on other fragments or degradation products of the β-amyloid precursor protein (APP). Particularly useful are antibodies which recognize a junction region within Aβ, where the junction region is located about the site of normal proteolytic cleavage of APP between residues Lys¹⁶ and Leu¹⁷ (Esch et al., Science, 248:1122-1124, 1990; Anderson et al., Neuroscience Lett., 128:126-128, 1991), typically spanning amino acid residues 13 and 28. Exemplary specific binding assays include two-site (sandwich) assays in which the capture antibody is specific for the junction region of Aβ, as just described, and a labeled second antibody is specific for an epitope other than the epitope recognized by the capture antibody. Particularly useful are second antibodies which bind to the amino-terminal end of Aβ, typically recognizing an epitope within amino acid residues 1-16. In another aspect, the present invention provides a system for detecting soluble Aβ in a fluid sample. The system includes a first binding substance, typically an antibody, specific for an epitope in a junction region of Aβ, as described above, and a second binding substance, typically an antibody, specific for an epitope of Aβ other than the epitope bound by the first binding substance. One of the first and second binding substances is bound to a solid phase, while the other is labeled, with the first binding substance preferably being a capture antibody bound to a solid phase and the second binding substance preferably being a labeled antibody, more preferably being an enzyme-labeled antibody. The system may further include substrate for the enzyme, the system is useful in performing enzyme-linked immunosorbent assays (ELISA) having high specificity and sensitivity for the detection of AD in fluid samples.

In certain preferred embodiments of the invention, Western blotting Aβ detection is used. In other preferred embodiments, ELISA (enzyme linked immunosorbent assay) Aβ detection is used. One description of such an embodiment is for example as follows: A monoclonal antibody (capture antibody, mAb 1) directed against the soluble antigen is adsorbed onto a solid substratum. The soluble antigen present in the sample binds to the antibody, and unreacted sample components are removed by washing. An enzyme-conjugated monoclonal antibody (detection antibody, mAb 2) directed against a second epitope of the antigen binds to the antigen captured by mAb 1 and completes the sandwich. After removal of unbound mAb 2 by washing, a substrate solution is added to the wells. In certain embodiments, a colored product is formed in proportion to the amount of antigen present in the sample. The reaction is terminated by addition of stop solution and absorbance may be measured spectrophotometrically or, in some embodiments, the product may be detected fluorometrically.

In preferred embodiments, the antibodies for use in the present invention are specific only for Aβ peptide.

In certain embodiments, the assay method of the present invention can be provided in the form of a kit, *e.g.*, a packaged combination of instructions for carrying out the assay, capture antibody, and solid support for immobilization as described hereinafter. In addition, a detection means may also be included, such as an antibody to the Aβ peptide, which may be labeled or unlabeled, as well as other additives, such as for example, stabilizers, washing, and incubation buffers, and the like.

Kits of the present invention, also will typically include a means for containing the reagents in close confinement for commercial sale such as, *e.g.*, injection or blow-molded plastic containers. Other containers suitable for conducting certain steps of the disclosed methods also may be provided.

### Definitions

The term "β-amyloid peptide" (Aβ) as used herein refers to an approximately 4.2 kD protein which, in the brains of subjects, *e.g.*, suffering from Alzheimer's disease (AD), Down's Syndrome, or HCHWA-D and some normal aged subjects, forms the subunit of the amyloid filaments comprising the senile (amyloid) plaques and the amyloid deposits in small cerebral and meningeal blood vessels (amyloid angiopathy). Aβ can occur in a filamentous polymeric form (in this form, it exhibits the Congo-red and thioflavin-S dye-binding characteristics of amyloid described in connection therewith). Aβ can also occur in a non-filamentous form ("preamyloid" or "amorphous" or "diffuse" deposits) in tissue, in which form no detectable birefringent staining by Congo red occurs. A portion of this protein in the insoluble form obtained from meningeal blood vessels is described in U.S. Patent No. 4,666,829. Aβ when used in connection with this invention, specifically refers to an approximately 39-43 amino acid peptide which can be found in and purified from the extracellular fluid (medium) of cultured cells grown *in vitro* or from body fluids of humans and other mammals, including both normal individuals and individuals suffering from Aβ-related conditions. However, amino truncated Aβ peptides, which are referred to as Aβₓ₋₄₀ have also been detected in the extracellular fluid (medium) by the assay used herein Thus, Aβ also refers to related Aβ sequences that result from mutations in the Aβ region of the normal gene. In whatever form, Aβ is an approximately 39-43 amino acid fragment, or Aβₓ₋₄₀, of a large membrane-spanning glycoprotein, referred to as the β-(amyloid precursor protein (APP), encoded by a gene on the long arm of human chromosome 21. Aβ is further characterized by its relative mobility in SDS-polyacrylamide gel electrophoresis or in high performance liquid chromatography (HPLC). Its 43-amino acid sequence is: 
AspAlaGluPheArgHisAspSerGlyTyrGlu ValHisHisGlnLysLeu ValPhePheAlaGluAsp ValGlySer
AsnLysGlyAlaIleIleGlyLeuMet ValGlyGly ValValIleAlaThr (SEQ ID NO: 1)
 or a sequence that is substantially homologous thereto.

The term "Aβ peptides" as used herein refers to intact or full length Aβ as well as to fragments and degradation products of Aβ which are generated at low concentrations by mammalian cells. Particular Aβ fragments have a molecular weight of approximately 3 kD and are presently believed to consist of amino acid residues 11-40 and 17-40 of Aβ.

The term "Aβ junction region" as used herein refers to a region of Aβ which is centered at the site between amino acid residues 16 and 17 (Lys¹⁶ and Leu¹⁷) which is a target for normal proteolytic processing of APP. Such normal processing results in a variety of APP fragments which are potentially immunologically cross-reactive with the intact Aβ molecule and fragments of Aβ which are to be identified in the methods of the present invention. The junction region will span amino acid residues 10 to 35, preferably spanning amino acid residues 15 to 30, with antibodies raised against a synthetic peptide consisting of amino acid residues 13-28 having been found to display the requisite specificity.

The term "β-amyloid precursor protein" or "APP" as used herein is defined as a polypeptide that is encoded by a gene of the same name localized in humans on the long arm of chromosome 21 and that includes Aβ within its carboxyl third. APP is a glycosylated, single-membrane-spanning protein expressed in a wide variety of cells in many mammalian tissues. Examples of specific isotypes of APP which are currently known to exist in humans are the 695-amino acid polypeptide described by Kang et al. (Nature, 325:733-736, 1987) which is designated as the "normal" APP; the 751-amino acid polypeptide described by Ponte et al. (Nature, 331:525-527, 1988) and Tanzi et al. (Nature, 331:528-530 1988); and the 770-amino acid polypeptide described by Kitaguchi et al. (Nature, 331:530-532 1988). Examples of specific variants of APP include point mutations which can differ in both position and phenotype (for review of known variant mutations see Hardy, Nature Genet., 1:233-234, 1992).

The term "APP fragments" as used herein refers to fragments of APP other than those which consist solely of Aβ or Aβ fragments. That is, APP fragments will include amino acid sequences of APP in addition to those which form intact Aβ or a fragment of Aβ.

The term "Aβ-related condition" or "amyloidopathy" as used herein is defined as including diffuse Lewis body disease, Creutzfeldt-Jakob disease, familial amyloidosis of Finnish type, familial amyloidotic polyneuropathy, and Hereditary cerebral hemorrhage with amyloidosis of the Dutch type,

The terms "conditioned culture medium" and "culture medium" as used herein refer to the aqueous extracellular fluid which surrounds cells grown in tissue culture (*in vitro*) and which contains, among other constituents, proteins and peptides secreted by the cells.

The term "body fluid" as used herein refers to those fluids of a mammalian host which will be expected to contain measurable amounts of Aβ and Aβ fragments, specifically including blood, cerebrospinal fluid (CSF), urine, and peritoneal fluid. The term "blood" refers to whole blood, as well as blood plasma and serum. According to the present invention, Aβ and Aβ fragments may be detected and/or measured in a variety of biological and physiological samples, including in vitro samples, such as conditioned medium from cultured cells, including transfected cell lines and endogenous cell lines, and in vivo patient samples, typically body fluids and brain tissue extracts. Detection and measurement of Aβ peptides may be accomplished by any technique capable of distinguishing Aβ and Aβ fragments from other APP fragments which might be found in the sample. Conveniently, immunological detection techniques may be employed using binding substances specific for Aβ, such as antibodies, antibody fragments, recombinant antibodies, and the like, which bind with specificity and sensitivity to Aβ. In particular, it has been found that antibodies which are monospecific for the junction region of Aβ are capable of distinguishing Aβ from other APP fragments. The junction region of Aβ is centered at amino acid residues 16 and 17, typically spanning amino acid residues 13-28, and such junction-specific antibodies may be prepared using synthetic peptides having that sequence as an immunogen. Particularly suitable detection techniques include ELISA, Western blotting, radioimmunoassay, and the like.

A preferred immunoassay technique is a two-site or "sandwich" assay employing a junction-specific antibody as the capture antibody (bound to a solid phase) and a second labeled antibody which binds to an epitope other than that bound to by the capture antibody. The second labeled antibody preferably recognizes the amino terminus of Aβ and may be conveniently raised against a synthetic peptide consisting essentially of amino acid residues 1-16 of Aβ.

Other non-immunologic techniques for detecting Aβ and Aβ fragments which do not require the use of Aβ specific antibodies may also be employed. For example, two-dimensional gel electrophoresis may be employed to separate closely related soluble proteins present in a fluid sample. Antibodies which are cross-reactive with many fragments of APP, including Aβ, may then be used to probe the gels, with the presence of Aβ being identified based on its precise position on the gel. In the case of cultured cells, the cellular proteins may be metabolically labeled and separated by SDS-polyacrylamide gel electrophoresis, optionally employing immunoprecipitation as an initial separation step.

*In vivo* detection of Aβ in patient samples can be used for monitoring the 1-aminocyclohexane derivative treatment of Alzheimer's Disease (AD) and other Aβ-related conditions according to the methods of the present invention. Suitable patient samples include body fluids, such as blood, CSF, urine, and peritoneal fluid. The presence of the Aβ-related condition will generally be associated with elevated levels of Aβ in the fluid when compared to those values in normal individuals, *i.e.*, individuals not suffering from AD or any other Aβ-related condition. Diagnostic concentrations of Aβ in blood are in the range from 0.1 ng/ml to 10 ng/ml or higher, more generally 0.1 ng/ml to 3 ng/ml. Diagnostic concentrations of Aβ in CSF are in the range from 0.1 ng/ml to 25 ng/ml or higher, more generally 0.1 ng/ml to 5 ng/ml.

The measured concentrations of Aβ may be monitored in order to follow the effectiveness of 1-aminocyclohexane derivative treatment. According to the invention, the levels of Aβ would decrease with an effective 1-aminocyclohexane derivative treatment regimen.

*In vitro* monitoring of Aβ levels in conditioned culture medium from a suitable cell culture may be used for screening methods of the invention. By growing cells under conditions which result in the accumulation of Aβ in the conditioned culture medium, and exposing the cultured cells to 1-aminocyclohexane derivatives, the effect of these 1-aminocyclohexane derivatives on Aβ production may be observed.

Suitable cell lines include human and animal cell lines, such as the 293 human kidney cell line, human neuroglioma cell lines, human HeLa cells, primary human endothelial cells (*e.g.*, HUVEC cells), primary human fibroblasts or lymphoblasts, primary human mixed brain cells (including neurons, astrocytes, and neuroglia), Chinese hamster ovary (CHO) cells, and the like. Preferred for use in the screening methods according to the present invention are cell lines capable of expressing APP variants which overproduce Aβ. By "overproduce," it is meant that the amount of Aβ produced from the variant APP will be greater than the amount produced from any or all of the normal APP isoforms, e.g., the 695, 751, and 770 amino acid isoforms which have been previously described. Particularly preferred are APP variants having one or several amino acid substitutions directly amino-terminal of the Aβ cleavage site. For example, K293 cells which express an APP DNA bearing a double mutation (Lys⁵⁹⁵ → Asn⁵⁹⁵ and Met⁵⁹⁶ → Leu⁵⁹⁶) found in a Swedish FAD family produce approximately six-to-eightfold more Aβ than cells expressing normal APP (*see* U.S. Patent Np. 5,593,846). The mutation at residue 596 appears to be principally responsible for the increase.

Similarly, in vivo monitoring of Aβ in animal models, such as the mouse animal model disclosed in WO 91/19810 and animal models expressing other APP isotypes and/or variants, may also be used to test the therapeutic effectiveness of various doses and regiments of 1-aminocyclohexane derivatives (usually starting with doses which have previously been identified by *in vitro* screens, such as the *in vitro* screens described above). The doses of 1-aminocyclohexane derivatives which reduce the level of the Aβ in certain body fluids are considered to be candidates for further evaluation.

The term "treat" is used herein to mean to relieve or alleviate at least one symptom of a disease in a subject. For example, in relation to amyloidopathy-associated dementia, the term "treat" may mean to relieve or alleviate cognitive impairment (such as impairment of memory and/or orientation) or impairment of global functioning (activities of daily living, ADL) and/or slow down or reverse the progressive deterioration in ADL or cognitive impairment. Within the meaning of the present invention, the term "treat" also denote to arrest, delay the onset (*i.e.*, the period prior to clinical manifestation of a disease) and/or reduce the risk of developing or worsening a disease. The term "protect" is used herein to mean prevent delay or treat, or all, as appropriate, development or continuance or aggravation of a disease in a subject. Within the meaning of the present invention, the disease is an amyloidopathy, which includes but is not limited to Alzheimer's disease (AD), Parkinson's disease, Down's syndrome, diffuse Lewis body disease, progressive supranuclear palsy, Creutzfeldt-Jakob disease, familial amyloidosis of Finnish type, familial amyloidotic polyneuropathy, Hereditary cerebral hemorrhage with amyloidosis of the Dutch type, and Gerstmann-Straussler Scheinker syndrome.

For example, as disclosed herein, a prophylactic administration of an 1-aminocyclohexane derivative can protect a recipient subject having elevated levels of fibrillogenic β-amyloid peptide (Aβ) (*e.g.*, individuals, who are homozygous or heterozygous mutants in one of several genes, such as the beta-amyloid precursor protein (APP), presenilins (PS1, PS2), secretases, such as β-amyloid cleaving enzyme (BACE), and apolipoprotein E; *see* also genetic screening and clinical analysis described in Goate, 1991, Nature, 349:704-706). Similarly, according to the present invention, a therapeutic administration of an 1-aminocyclohexane derivative can lead to slow-down in the development of clinical symptoms or even regression of symptoms.

Within the meaning of the present invention, the term "NMDA antagonist drugs" is used to refer to drugs that can suppress the normal triggering of NMDA receptor-mediated neuronal firings. Preferred NMDA antagonist drugs of the invention are 1-aminocyclohexane derivatives such as memantine and neramexane. These compounds also have 5HT₃ antagonist activity and/or neuronal nicotinic receptor antagonist activity.

The term "analog" or "derivative" is used herein in the conventional pharmaceutical sense, to refer to a molecule that structurally resembles a reference molecule (such as 1-aminocyclohexane), but has been modified in a targeted and controlled manner to replace one or more specific substituents of the referent molecule with an alternate substituent, thereby generating a molecule which is structurally similar to the reference molecule. Synthesis and screening of analogs (*e.g.*, using structural and/or biochemical analysis), to identify slightly modified versions of a known compound which may have improved or biased traits (such as higher potency and/or selectivity at a specific targeted receptor type, greater ability to penetrate mammalian blood-brain barriers, fewer side effects, etc.) is a drug design approach that is well known in pharmaceutical chemistry.

The term "1-aminocyclohexane derivative" is used herein to describe a compound which is derived from 1-aminocyclohexane (or an available derivative thereof, such as neramexane or memantine) in the process used to create a similar but slightly different drug.

The 1-aminocyclohexane derivatives of the present invention can be represented by the general formula (I): 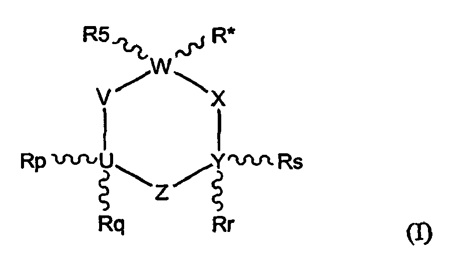 wherein:
- R* is -(A)ₙ-(CR¹R²)ₘ-NR³R⁴,
   n+m = 0, 1, or 2,
   A is selected from the group consisting of linear or branched lower alkyl (C₁-C₆),linear or branched lower alkenyl (C₂-C₆), and linear or branched lower alkynyl (C₂-C₆),
   R¹ and R² are independently selected from the group consisting of hydrogen, linear or branched lower alkyl (C₁-C₆), linear or branched lower alkenyl (C₂-C₆), linear or branched lower alkynyl (C₂-C₆) aryl, substituted aryl and arylalkyl,
   R³ and R⁴ are independently selected from the group consisting of hydrogen, linear or branched lower alkyl (C₁-C₆), linear or branched lower alkenyl (C₂-C₆), and linear or branched lower alkynyl (C₂-C₆), or together form alkylene (C₂-C₁₀) or alkenylene (C₂-C₁₀) or together with the N form a 3-7-membered azacycloalkane or azacycloalkene, including substituted (alkyl (C₁-C₆), alkenyl (C₂-C₆)) 3-7-membered azacycloalkane or azacycloalkene; or independently R³ or R⁴ may join with R^{p}, R^{q}, R^{r}, or R^{s} to form an alkylene chain -CH(R⁶)-(CH₂)ₜ-, wherein t= 0 or 1 and the left side of the alkylene chain is attached to U or Y and the right side of the alkylene chain is attached to N and R⁶ is selected from the group consisting of hydrogen, linear or branched lower alkyl (C₁-C₆), linear or branched lower alkenyl (C₂-C₆), linear or branched lower alkynyl (C₂-C₆), aryl, substituted aryl and arylalkyl; or independently R³ or R⁴ may join with R⁵ to form an alkylene chain represented by the formula -CH₂-CH₂-CH₂-(CH₂)ₜ-, or an alkenylene chain represented by the formulae -CH=CH-CH₂-(CH₂)ₜ-, - CH=C=CH-(CH₂)ₜ- or -CH₂-CH=CH-(CH₂)ₜ-, wherein t= 0 or 1, and the left side of the alkylene or alkenylene chain is attached to W and the right side of the alkylene ring is attached to N;
- R⁵ is independently selected from the group consisting of hydrogen, linear or branched lower alkyl (C₁-C₆), linear or branched lower alkynyl (C₂-C₆), and linear or branched lower alkynyl (C₂-C₆), or R⁵ combines with the carbon to which it is attached and the next adjacent ring carbon to form a double bond,
- R^{p}, R^{q}, R^{r}, and R^{s}, are independently selected from the group consisting of hydrogen, linear or branched lower alkyl (C₁-C₆), linear or branched lower alkenyl (C₂-C₆), linear or branched lower alkynyl (C₂-C₆), cycloalkyl (C₃-C₆) and aryl, substituted aryl and arylaklyl or R^{P}, R^{q}, R^{r}, and R^{s} independently may form a double bond with U or with Y or to which it is attached, or R^{P}, R^{q}, R^{r}, and R^{s} may combine together to represent a lower alkylene -(CH₂)ₓ- or a lower alkenylene bridge wherein x is 2-5, inclusive, which alkylene bridge may, in turn, combine with R⁵ to form an additional lower alkylene
- (CH₂)_{y}- or a lower alkenylene bridge, wherein y is 1-3, inclusive,
- the symbols U, V, W, X, Y, Z represent carbon atoms,
and include optical isomers, diastereomers, polymorphs, enantiomers, hydrates, pharmaceutically acceptable salts, and mixtures of compounds within formula (I).

The ring defined by U-V-W-X.Y-Z is preferably selected from the group consisting of cyclohexane, cyclohex-2-ene, cyclohex-3-ene, cyclohex-1,4-diene, cyclohex-1,5-diene, cyclohex-2,4-diene, and cyclohex-2,5-diene,

Non-limiting examples of 1-aminocyclohexane derivatives used according to the invention include the 1-aminoalkylcyclohexane derivatives selected from the group consisting of:
1-amino-1,3,5-trimethylcyclohexane,
1-amino-1 (trans),3(trans),5-trimethylcyclohexane,
1-amino-1 (cis),3 (cis), 5-trimethylcyclohexane,
1-amino-1,3,3,5-tetramethylcyclohexane,
1-amino-1,3,3,5,5-pentamethylcyclohexane (neramexane),
1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcylohexane,
1-amino-1-ethyl-3,3,5,5-tetramethylcyclohexane,
1-amino-1-propyl-3,3,5,5-tetramethylcyclohexane,
N-methyl-1-amino-1,3,3,5,5-pentamethylcyclohexane,
N-ethyl-1-amino-1,3,3,5,5-pentamethyl-cyclohexane,
N-(1,3,3,5,5-pentamethylcyclohexyl) pyrrolidine,
3,3,5,5-tetramethylcyclohexylmethylamine,
1-amino-1-propyl-3,3,5,5-tetramethylcyclohexane,
1 amino-1,3,3,5(trans)-tetramethylcyclohexane (axial amino group),
3-propyl-1,3,5,5-tetramethylcyclohexylamine semihydrate,
1-amino-1,3,5,5-tetramethyl-3-ethylcyclohexane,
1-amino-1,3,5-trimethylcyclohexane,
1-amino-1,3-dimethyl-3-propylcyclohexane,
1-amino-1,3(trans),5(tans)-trimethyl-3(cis)-propylcyclohexane,
1-amino-1,3-dimethyl-3-ethylcyclohexane,
1-amino-1,3,3-trimethylcyclohexane,
cis-3-ethyl-1(trans)-3(trans)-S-trimethylcyclohexamine,
1-amino-1,3(trans)-dimethylcyclohexane,
1,3,3-trimethyl-S,S-dipropylcyclohexylamine,
1-amino-1-methyl-3 (trans)-propylcyclohexane,
1-methyl-3 (cis)-propylcyclohexylamine,
1-amino-1-methyl-3(trans)-ethylcyclohexane,
1-amino-1,3,3-trimethyl-5(cis)-ethylcyclohexane,
1-amino-1,3,3-trimethyl-5(trans)-ethylcyclohexane,
cis-3-propyl-1,5,5-trimethylcyclohexylamine,
trans-3-propyl-1,5,5-trimethylcyclohexylamine,
N-ethyl-1,3,3,5,5-pentamethylcyclohexylamine,
N-methyl-1-amino-1,3,3,5.5-pentamethylcyclohexane,
1-amino-1-methylcyclohexane,
N,N-dimethyl-1-amino-1,3,3,5,5-pentamethylcyclohexane,
2-(3,3,5,5-tetramethylcyclohexyl)ethylamine,
2-methyl-1-(3,3,5,5-tetramethylcyclohexyl)propyl-2-amine,
2-(1,3,3,5,5-pentamethylcyclohexyl-1)-ethylamine semihydrate,
N-(1,3,3,5,5-pentamethylcyclohexyl)-pyrrolidine,
1-amino-1,3(trans),5(trans)-trimethylcyclohexane,
1-amino-1,3(cis),5(cis)-timethylcyclohexane,
1-amino-(1R,SS)trans-S-ethyl-1,3,3-trimethylcyclohexane,
1-amino-(1 S ,SS)cis- 5 -ethyl-1,3 ,3-trimethylcyclohexane,
1-amino-1,5, 5-trimethyl-3(cis)-isopropyl-cyclohexane,
1-amino-1,5,5-trimethyl-3(trans)-isopropyl-cyclohexane,
1-amino-1-methyl-3(cis)-ethyl-cyclohexane,
1-amino-1-methyl-3(cis)-methyl-cyclohexane,
1-amino-5,5-diethyl-1,3,3-trimethyl-cyclohexane,
1-amino-1,3,3,5,5-pentamethylcyclohexane,
1-amino-1,5,5-trimethyl-3,3-diethylcyclohexane,
1-amino-1-ethyl-3,3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylcyclohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,SS)cis-5-ethyl-1,3,3-trimethylcyclohexyl]pyrrolidine or piperidine,
N-(1,3,3-trimethyl-trans-5-ethylcyclohexyl)pyrrolidine or piperidine,
N-[(1R,SS)trans-5-ethyl,3,3-trimethylcyclohexyl]pyrrolidine or piperidine,
N-(1-ethyl-3,3,5,5-tetramethylyclohexyl)pyrrolidine or piperidine,
N-(1-propyl-3,3,5,5-tetramethylcyclohexyl)pyrrolidine or piperidine,
N-(1,3,3,5,5-pentamethylcyclohexyl)pyrrolidine,
their optical isomers, diastereomers, enantiomers, hydrates, their pharmaceutically acceptable salts, and mixtures thereof.

Neramexane (1-amino-1,3,3,5,5-pentamethylcyclohexane) is disclosed, *e.g.*, in U.S. Patent Application No. 09/597,102 and U.S. Patent No. 6,034,134.

Certain 1-aminocyclohexane derivatives of general formula (I) including the case where three axial alkyl substituent, *e.g.*, R^{p}, R^{r} and R⁵ all together form a bridgehead to yield compounds (so called 1-aminoadamantanes) illustrated by the formulae IIb - IId below: 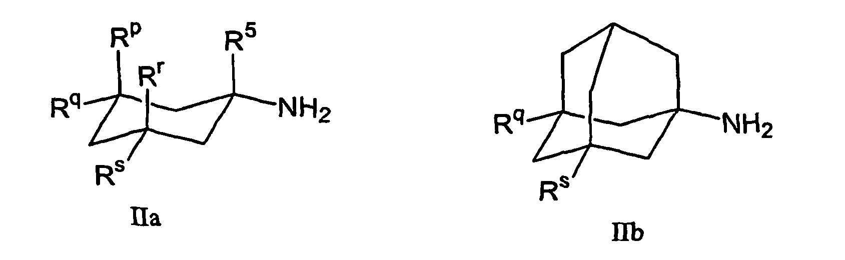 or

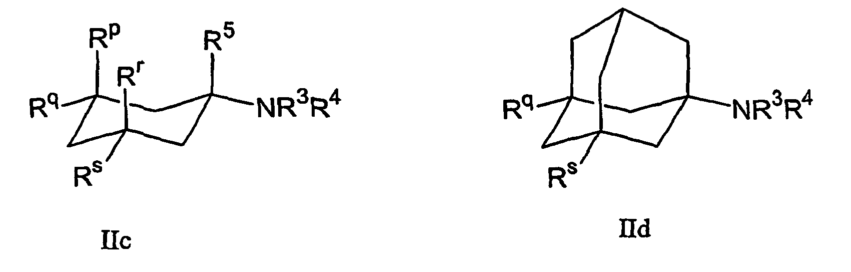

Certain 1-aminocyclohexane derivatives of forumula (I) wherein n + m = 0, U, V, W, X, Y and Z form a cyclohexane ring, and one or both of R³ and R⁴ are independently joined to said cyclohexane ring via alkylene bridges formed through R^{p}, R^{q}, R^{r}, R^{s} or R⁵ are represented by the following formulae IIIa-IIIc: 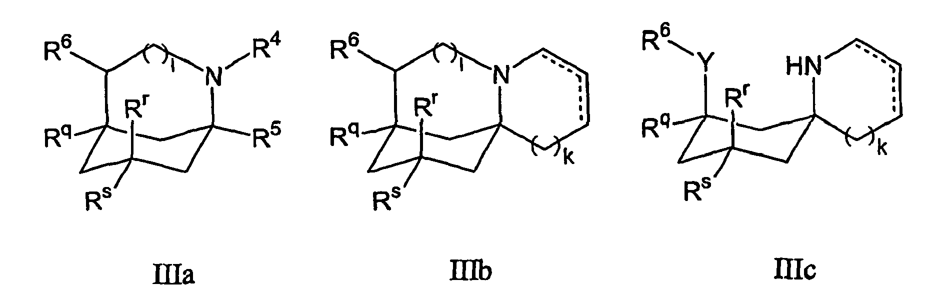 where R^{q}, R^{r}, R^{s}, R^{r} and R⁵ are as defined above for formula (I), R⁶ is hydrogen, linear or branched lower alkyl (C₁-C₆), linear or branched lower alkenyl (C₂-C₆), linear or branched lower alkynyl (C₂-C₆), aryl, substituted aryl or arylalkyl Y is saturated or may combine with R⁶ to form a carbon-hydrogen bond with the ring carbon to which it is attached, l= 0 or 1 and k= 0, 1 or 2 and ----- represents a single or double bond.

Non-limiting examples of 1-aminocyclohexane derivatives used according to the invention include 1-amino adamantane and its derivatives selected from the group consisting of:
1-amino-3-phenyl adamantane,
1-amino-methyl adamantane,
1-amino-3,5-dimethyl adamantane (memantine),
1-amino-3-ethyl adamantane,
1-amino-3-isopropyl adamantane,
1-amino-3-n-butyl adamantane,
1-amino-3,5-diethyl adamantane,
1-amino-3,5-diisopropyl adamantane,
1-amino-3,5-di-n-butyl adamantane,
1-amino-3-methyl-5-ethyl adamantane,
1-N-methylamino-3,5-dimethyl adamantane,
1-N-ethylamino-3,5-dimethyl adamantane,
1-N-isopropyl-amino-3,5-dimethyl adamantane,
1-N,N-dimethyl-amino-3,5-dimethyl adamantane,
1-N-methyl-N-isopropyl-amino-3-methyl-5-ethyl adamantane,
1-amino-3-butyl-5-phenyl adamantane,
1-amino-3-pentyl adamantane,
1-amino-3,5-dipentyl adamantane,
1-amino-3-pentyl-5-hexyl adamantane,
1-amino-3-pentyl-5-cyclohexyl adamantane,
1-amino-3-pentyl-5-phenyl adamantane,
1-amino-3-hexyl adamantane,
1-amino-3,5-dihexyl adamantane,
1-amino-3-hexyl-5-cyclohexyl adamantane,
1-amino-3-hexyl-5-phenyl adamantane,
1-amino-3-cyclohexyl adamantane,
1-amino-3,5-dicyclohexyl adamantane,
1-amino-3-cyclohexyl-5-phenyl adamantane,
1-amino-3,5-diphenyl adamantane,
1-amino-3,5,7-trimethyl adamantane,
1-amino-3,5-dimethyl-7-ethyl adamantane,
1-amino-3,5-diethyl-7-methyl adamantane,
1-N-pyrrolidino and 1-N-piperidine derivatives,
1-amino-3-methyl-5-propyl adamantane,
1-amino-3-methyl-5-butyl adamantane,
1-amino-3-methyl-5-pentyl adamantane,
1-amino-3-methyl-5-hexyl adamantane,
1-amino-3-methyl-5-cyclohexyl adamantane,
1-amino-3-methyl-5-phenyl adamantane,
1-amino-3-ethyl-5-propyl adamantane,
1-amino-3-ethyl-5-butyl adamantane,
1-amino-3-ethyl-5-pentyl adamantane,
1-amino-3-ethyl-5-hexyl adamantane,
1-amino-3-ethyl-5-cyclohexyl adamantane,
1-amino-3-ethyl-5-phenyl adamantane,
1-amino-3-propyl-5-butyl adamantane,
1-amino-3-propyl-5-pentyl adamantane,
1-amino-3-propyl-5-hexyl adamantane,
1-amino-3-propyl-5-cyclohexyl adamantane,
1-amino-3-propyl-5-phenyl adamantane,
1-amino-3-butyl-5-pentyl adamantane,
1-amino-3-butyl-5-hexyl adamantane,
1-amino-3-butyl-5-cyclohexyl adamantane,
their optical isomers, diastereomers, enantiomers, hydrates, N-methyl, N,N-dimethyl, N-ethyl, N-propyl derivatives, their pharmaceutically acceptable salts, and mixtures thereof.

### Additional Preferred Compounds of the Invention

Preferred 1-aminocyclohexane derivatives used according to the invention include the 1-aminocyclohexane derivatives of the formula 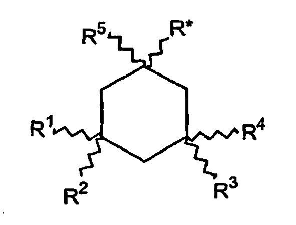 wherein R* is -(CH₂)*ₙ*(CR⁶R⁷)*ₘ*-NR⁸R⁹
wherein n+m=0, 1, or 2
wherein R¹ through R⁷ are independently selected from hydrogen and lower-alkyl (1-6C), at least R¹, R⁴, and R⁵ being lower-alkyl, and wherein R⁸ and R⁹ are independently selected from hydrogen and lower-alkyl (1-6C) or together represent lower-alkylene -(CH₂)*ₓ*- wherein x is 2 to 5, inclusive, and enantiomers, optical isomers, hydrates, and pharmaceutically-acceptable salts thereof.

Preferred 1-aminocyclohexane derivatives used according to the invention also include the 1-aminocyclohexane derivatives of the formula 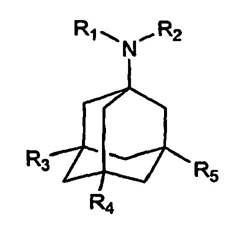 wherein R₁ and R₂ are identical or different and represent hydrogen or a straight or branched alkyl group of 1 to 6 C atoms or, in conjunction with N, a heterocyclic group with 5 or 6 ring C atoms;
wherein R₃ and R₄ are identical or different, being selected from hydrogen, a straight or branched alkyl group of 1 to 6 C atoms, a cycloalkyl group with 5 or 6 C atoms, and phenyl;
wherein R₅ is hydrogen or a straight or branched C₁-C₆ alkyl group,
or a pharmaceutically-acceptable salt thereof.

Preferred 1-aminocyclohexane derivatives used according to the invention also include the 1-aminocyclohexane derivatives of the formula 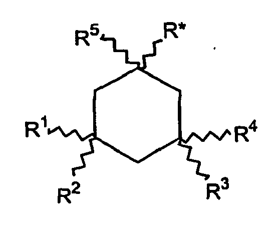 wherein R* is -(CH₂)*ₙ*-(CR⁶R⁷)*ₘ*-NR⁸R⁹
wherein n+m=0,1, or 2
wherein R¹ through R⁷ are independently selected from hydrogen, straight or branched lower-alkyl (1-6C), -CH₂-, and lower-cycloalkyl (1-6C), at least R¹, R⁴, and R⁵ being lower-alkyl or -CH₂-, and
wherein R⁸ and R⁹ are independently selected from hydrogen, straight or branched lower-alkyl (1-6C), and lower-cycloalkyl (1-6C), or together represent lower-alkylene -(CH₂)ₓ- wherein x is 2 to 5, inclusive, or, in conjunction with N, represent a heterocyclic group with 5 or 6 ring C atoms;
provided that when R¹, R⁴, and R⁵ are each independently -CH₂-
R¹, R⁴, and R⁵ are each bonded to a single CR^{a} group to form a bridge, wherein R^{a} is selected from hydrogen, a straight or branched lower alkyl group (1-6C), a cycloalkyl group (5-6C), and phenyl;
R² is selected from hydrogen, a straight or branched lower alkyl group (1-6C), a cycloalkyl group (5-6C), and phenyl;
R³ is hydrogen or a straight or branched lower alkyl group (1-6C); and
R* is -(CH₂)*ₙ*-(CR⁶R⁷)*ₘ*-NR⁸R⁹, wherein n+m=0, and R⁸ and R⁹ are identical or different and represent hydrogen or a straight or branched lower alkyl group (1-6C) or, in conjunction with N, a heterocyclic group with 5 or 6 ring C atoms; and enantiomers, optical isomers, hydrates, and pharmaceutically-acceptable salts thereof.

Memantine (1-amino-3,5-dimethyl adamantane), for example, is the subject matter of U.S. Patents No. 4,122,193 and 4,273,774.

The 1-amino adamantane derivatives of formulae IIb and IId, including memantine, are generally prepared by alkylation of halogenated adamantanes, preferably bromo- or chloroadamantanes. The di- or tri-substituted adamantanes are obtained by additional halogenation and alkylation procedures. The amino group is introduced either by oxidation with chromiumtrioxide and bromination with HBr or bromination with bromine and reaction with formamide followed by hydrolysis. The amino function can be alkylated according to generally-accepted methods. Methylation can, for example, be effected by reaction with chloromethyl formate and subsequent reduction. The ethyl group can be introduced by reduction of the respective acetamide. For more details on synthesis *see*, *e.g.*, U.S. Patents No. 5,061,703 and 6,034,134. Additional synthetic techniques for the foregoing compounds can be found in provisional applications Ser. No. 60/350,974 filed November 7, 2001, Ser. No. 60/337,858 filed November 8, 2001, and Ser. No. 60/366,386 filed March 21,2002, all incorporated by reference, as well as in the Synthesis Examples below.

According to the invention, the 1-aminocyclohexane derivatives of formula (I) may be applied as such or used in the form of their pharmaceutically-acceptable salts including, for example, the acid addition salts such as hydrochlorides, hydrobromides, sulfates, acetates, succinates or tartrates, or their acid addition salts with fumaric, maleic, citric, or phosphoric acids.

In addition, using methods known to those skilled in the art, analogs and derivatives of the compounds of the invention can be created which have improved therapeutic efficacy in controlling dementia, *i.e.*, higher potency and/or selectivity at a specific targeted receptor type, either greater or lower ability to penetrate mammalian blood-brain barriers (*e.g.*, either higher or lower blood-brain barrier permeation rate), fewer side effects, etc.

Various salts and isomers (including stereoisomers and enantiomers) of the drugs listed herein can be used. The term "salts" can include addition salts of free acids or free bases. Examples of acids which may be employed to form pharmaceutically acceptable acid addition salts include inorganic acids such as hydrochloric, sulfuric, or phosphoric acid, and organic acids such as acetic, maleic, succinic, or citric acid, etc. All of these salts (or other similar salts) may be prepared by conventional means. The nature of the salt or isomer is not critical, provided that it is non-toxic and does not substantially interfere with the desired pharmacological activity.

The term "therapeutically effective" applied to dose or amount refers to that quantity of a compound or pharmaceutical composition that is sufficient to result in a desired activity upon administration to a mammal in need thereof. As used herein with respect to the pharmaceutical compositions comprising an 1-aminocyclohexane derivative, the term "therapeutically effective amount/dose" is used interchangeably with the term "neurologically effective amount/dose" and refers to the amount/dose of a compound or pharmaceutical composition that is sufficient to produce an effective neurological response upon administration to a mammal. Note that when a combination of active ingredients is adminstered the effective amount of the combination may or may not include amounts of each ingredient that are individually effective.

The term "subthreshold" referring to the amount of an active ingredient means an amount inadequate to produce a response, *i.e*., an amount below the minimum effective amount. The term "suboptimal" in the same context means an amount of an active ingredient that produces a response but not to its full extent, which would be achieved with a higher amount.

The phrase "pharmaceutically acceptable", as used in connection with compositions of the invention, refers to molecular entities and other ingredients of such compositions that are physiologically tolerable and do not typically produce untoward reactions when administered to a mammal (*e.g.*, human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical compositions of the invention refers to a diluent, excipient, or vehicle with which an active compound (*e*.*g*., an 1-aminocyclohexane derivative and/or an AChED is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by E.W. Martin, 18^{th} Edition.

The term "subject" as used herein refers to a mammal (*e.g.*, rodent such as mouse or rat). In particular, the term refers to humans.

As used herein, the term "body fluid" refers to a biological sample of liquid containing the Aβ peptide. Such fluids include aqueous fluids such as serum, plasma, lymph fluid, synovial fluid, follicular fluid, seminal fluid, amniotic fluid, milk, whole blood, urine, cerebro-spinal fluid, saliva, sputum, tears, perspiration, mucus, tissue culture medium, tissue extracts, and cellular extracts.

The term "about" or "approximately" usually means within 20%, more preferably within 10%, and most preferably still within 5% of a given value or range. Alternatively, especially in biological systems, the term "about" means within about a log (*i.e*., an order of magnitude) preferably within a factor of two of a given value.

### Antibodies and Immunodetection Methods

As used herein, the term "antibodies" includes all types of immunoglobulin molecules, monoclonal antibodies, polyclonal antibodies, affinity-purified polyclonal antibodies, Fab and (Fab)₂, single-chain (SC) antibodies, or other molecules which specifically bind an epitope on Aβ. Such antibodies are produced in accordance with known techniques in the art. Generally, the antibodies used to detect Aβ according to this invention will be labeled with a detectable label, such as a radiolabel, a fluorescent label, second antibody specific for a separate epitope on Aβ antibody where the second antibody is conjugated to an enzyme that is used to catalyze the production of a detectable signal.

Any antibody which specifically binds to an epitope on Aβ is potentially useful in the assays of this invention. Examples of such antibodies include for example and without limitation, two antibodies (pAb 1-17 and pAb 17-28) to residues 1-17 and 17-28 of Aβ made by Quality Controlled Biochemicals Inc. (Hopkinton, Mass.), and Ab 6E10 to Aβ 1-17 and mAb 4G8 to Aβ 17-24, commercially available from Senetek, and antibodies described in U.S. Pat. No. 5,955,317, to Suzuki et al, the disclosure of which is hereby incorporated by reference. Many suitable techniques for using such antibodies to detect Aβ epitopes will be apparent to the skilled artisan, including fluorescence activated cell sorting (FACS), sandwich assays, competitive immunoassays, ELISA assays, Western blots, dot blots, ouchterlony plates, immunoelectrophoresis, fluorimetry, microcopy, fluorescence microscopy, ultra-filtration (using radiolabeled antibodies) and others.

In a preferred embodiments, the body fluid is analyzed by ELISA using antibodies specific for epitopes on Aβ. An ELISA apparatus typically comprises a 96 well microtiter plate, the inside surfaces of which are coated with one of the Aβ-specific antibodies. This coating, binding or attachment of the antibody to the solid phase is not a chemical reaction but rather is believed to result from a physical or noncovalent interaction between the polystyrene matrix of the microtiter plate and the antibody. A sample suspected of containing the target molecule Aβ is placed in contact with the coated microtiter plate so that binding will occur between the ligand A.beta. in the sample and the antibody. Any unbound components in the sample fluid are then removed from the plate wells by several washing steps. A second antibody which specifically recognizes the target molecule and is linked to a signal-generating enzyme is then added. Detection of the enzyme which is indicative of the presence of the target molecule in the sample is typically performed by addition of reagents which produce a detectable signal such as fluorescence or a color change.

In accordance with the present invention, preferably the body fluid is contacted and incubated with an immobilized capture antibody. The solid phase used for immobilization may be any inert support or carrier that is preferably water insoluble and useful in immunometric assays, including supports in the form of, *e.g.*, surfaces, particles, porous matrices, etc. Examples of commonly used supports include small sheets, Sephadex, polyvinyl chloride, plastic beads, and assay plates or test tubes manufactured from polyethylene, polypropylene, polystyrene, and the like including 96-well microtiter plates, as well as particulate materials such as filter paper, agarose, cross-linked dextran, and other polysaccharides. Alternatively, reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 are suitably employed for capture reagent immobilization. The preferred solid phase used is a multi-well microtiter plate that can be used to analyze several samples at one time. The most preferred is a microtest 96-well ELISA plate such as that sold as Nunc Maxisorb or Immulon. The solid phase is coated with the capture reagent as defined above, which may be linked by a non-covalent or covalent interaction or physical linkage as desired. If 96-well plates are utilized, they are preferably coated with the capture antibody and incubated for at least about 10 hours, more preferably at least overnight.

The coated plates are then typically treated with a blocking agent that binds non specifically to and saturates the binding sites to prevent unwanted binding of the detection antibody to the excess non-specific sites on the surfaces of the wells of the plate. Examples of appropriate blocking agents for this purpose include, *e.g.*, gelatin, bovine serum albumin, egg albumin, casein, and non-fat milk. After coating and blocking, the body fluid to be analyzed, appropriately diluted, is added to the immobilized phase.

The conditions for incubation of sample and immobilized capture reagent are selected to optimize sensitivity of the assay. Usually constant temperatures are maintained during the incubation period. Various buffers may be employed to achieve and maintain the desired pH during this step, including borate, phosphate, carbonate, Tris-HCl or Tris-phosphate, citrate, acetate, barbital, and the like. The particular buffer employed is not critical to the invention, but in individual assays one buffer may be preferred over another.

The body fluid is separated (preferably by washing) from the immobilized capture antibody to remove uncaptured body fluid. The solution used for washing is generally a buffer ("washing buffer") with a pH that will depend on the capture reagent utilized. The washing may be done one or more times.

In the last step of the assay method, the Aβ that is now bound to the capture antibody is measured. This measurement may be accomplished by many techniques, such as extraction to remove the bound Aβ from the capture reagent followed by bioassay, radioreceptor assay, or radioimmunoassay.

More preferably, however, the amount of free ligand is analyzed in the same plate, without the need for extraction or other cumbersome steps, using a standard ELISA method as detection means. In this procedure, preferably a molar excess of an antibody with respect to the maximum concentration of Aβ expected is added to the plate after it is washed.

The detection antibody added to the immobilized capture antibody will be either directly labeled, or detected indirectly by addition, after washing off of excess first antibody, of a molar excess of a second, labeled antibody directed against the first detection antibody.

The label used for either the first or second detection antibody is any detectable functionality that does not interfere with the binding of free ligand to the antibody. Examples of suitable labels are those numerous labels known for use in immunoassay, including moieties that may be detected directly, such as fluorochrome, chemiluminscent, and radioactive labels, as well as moieties, such as enzymes, that must be reacted or derivatized to be detected. Examples of such labels include the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, *e.g.*, firefly luciferase and bacterial luciferase (U.S. Pat. No. 4,737,456), luciferin, 2,3-dihydrophthalazinediones, horseradish peroxidase (HRP), alkaline phosphatase, β-galactosidase, glucoamylase, lysozyme, saccharide oxidases, *e.g.*, glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

Conventional methods are available to bind these labels covalently to proteins or polypeptide. Preferred labels herein are enzymes such as horseradish peroxidase and alkaline phosphatase.

Following the addition of last labeled antibody, the amount of bound antibody is determined by removing excess unbound labeled antibody through washing and then measuring the amount of the attached label using a detection method appropriate to the label, and correlating the measured amount with the amount of Aβ in the body fluid.

As a matter of convenience, the assay method of this invention can be provided in the form of a kit, *i.e*., a packaged combination of instructions for carrying out the assay, capture reagent as defined above, antibodies, standards for the Aβ, and solid support for immobilization as defined above. In addition, a detection means as defined above may be included, such as a specific antibody to the Aβ, which is labeled or unlabeled, as well as other , additives such as stabilizers, washing and incubation buffers, and the like.

### Cell Line Assays

Suitable cell lines for the assays of the invention include various human and animal cell lines which synthesize, process and secrete amyloid peptides, such as human neuroblastoma cell lines (*e.g.*, SK-N-SH), human neuroglioma cell lines, human HeLa cells, human kidney cell line HEK-293, primary human endothelial cells (*e.g*., HUVEC cells), primary human fibroblasts or lymphoblasts, primary human mixed brain cells (including neurons, astrocytes, and neuroglia), Chinese hamster ovary (CHO) cells, and the like. Preferred for use according to the present invention are human cell lines that express APP variants or that overproduce Aβ, *e.g.*, APP variants having one or several amino acid substitutions directly at the N-terminus of the Aβ cleavage site (*e*.*g*., K293 cells which express an APP DNA bearing a double mutation [Lys⁵⁹⁵ → Asn⁵⁹⁵ and Met⁵⁹⁶→Leu⁵⁹⁶] found in a Swedish FAD family, which produce approximately six-to-eight-fold more Aβ than cells expressing normal APP, as disclosed in the U.S. Patent No. 6,284,221).

Amyloid peptides produced by the cell lines before and after treatment with an 1-aminocyclohexane derivative may be detected using immunodetection methods outlined above.

To measure extracellular sAPP and fragments thereof, the cell culture supernatant can be utilized. To measure the full-length intracellular APP and fragments thereof, the cell lysates can be utilized. For example, to measure cell-associated full-length APP or to measure carboxyl-terminal fragments of APP, cell lysates can be incubated with antibody which recognizes the carboxyl-terminus of APP (*see, e.g.,* Buxbaum et al., Proc. Natl. Acad. Sci. USA, 87:6003-6, 1990). To measure Aβ peptides, or to measure sAPP, which is the secreted carboxyl-terminal truncated form, cell supernatants can be incubated with antibody, which recognizes the first 15 amino acids of the Aβ that correspond to the COOH-terminal amino acids of sAPP (*see*, *e.g.*, Buxbaum et al., Proc. Natl. Acad. Sci. USA, 91:4489-93, 1994).

The amount of extracellular Aβ peptides and the amount of extracellular sAPP can be normalized to the amount of total APP found in the cell. This normalization provides an effective means of accounting for any differences between cultures and any differences due to altered APP synthesis or maturation in cells treated with1-aminocyclohexane derivatives.

### Therapeutic Evaluations

The criteria for the diagnosis of Alzheimer's Disease (AD) is well known and is set forth in the guidelines of the National Institute of Neurological and Communicative Disorders and Alzheimer's Disease and Related Disorders Association (McKhann et al., Neurology, 34: 939-944, 1984); and in the American Psychiatric Association, Diagnostic and Statistical Manual of Mental Disorders (Diagnostic and Statistical Manual IV), all of which are incorporated herein by reference. Generally the objective criteria for the diagnosis of AD include: gradual memory impairment and gradual onset of at least one of the following aphasia, apraxia, agnosia or disturbance of executive functioning.

Treatment may be continued until there is a reduction in the symptoms of AD and the dosage may be adjusted in response to the mammal's individual response. Generally a positive response will not be expected until therapy has been continued for a minimum period of 90 to 365 days.

### Pharmaceutical Compositions

In conjunction with the methods of the present invention, also provided are pharmaceutical compositions comprising a therapeutically effective amount of an 1-aminocyclohexane derivative (such as memantine or neramexane) as well as, optionally, an additional carrier or excipient (all pharmaceutically acceptable). The compositions can be formulated for once-a-day administration or twice-a-day administration.

In the disclosed compositions, preferably, the 1-aminocyclohexane derivative is present in a therapeutically effective amount. The optimal therapeutically effective amount should be determined experimentally, taking into consideration the exact mode of administration, form in which the drug is administered, the indication toward which the administration is directed, the subject involved (*e.g*., body weight, health, age, sex, etc.), and the preference and experience of the physician or veterinarian in charge. As disclosed herein, for human administration, the 1-aminocyclohexane derivatives are administered in suitable form in doses ranging from about 1 to 100 mg per day. More specifically, the 1-aminocyclohexane derivatives are preferably administered at doses 5-60 mg/day, and especially 10-40 mg/day. It may also be desirable in certain cases to administer the active ingredient in a suboptional or subthreshold amount, and such administration would also be within the invention.

The invention also provides a method for preparing pharmaceutical compositions comprising admixing an 1-aminocyclohexane derivative and optionally one or more physiologically acceptable carriers and/or excipients and/or auxiliary substances.

In therapeutic applications, the pharmaceutical compositions are administered to a host already suffering from the disease. The pharmaceutical compositions will be administered in an amount sufficient to inhibit further deposition of Aβ plaque. An amount adequate to accomplish this is defined as a "therapeutically effective dose."

For prophylactic applications, the pharmaceutical compositions of the present invention are administered to a host susceptible to the Aβ-related disease, but not already suffering from such disease. Such hosts may be identified by genetic screening and clinical analysis, as described in the medical literature (*e.g*.*,* Goate, Nature, 349:704-706, 1991). The pharmaceutical compositions will be able to inhibit or prevent deposition of the Aβ plaque at a symptomatically early stage, preferably preventing even the initial stages of the β-amyloid disease. The amount of the compound required for such prophylactic treatment, referred to as a prophylactically-effective dosage, is generally the same as described for therapeutic treatment.

### Administration

The active agents of the present invention may be administered orally, topically, parenterally, or mucosally (*e.g.*, buccally, by inhalation, or rectally) in dosage unit formulations containing conventional non-toxic pharmaceutically acceptable carriers. It is usually desirable to use the oral route. The active agents may be administered orally in the form of a capsule, a tablet, or the like (*see* Remington's Pharmaceutical Sciences, Mack 5 Publishing Co., Easton, PA). The orally administered medicaments may be administered in the form of a time-controlled release vehicle, including diffusion-controlled systems, osmotic devices, dissolution-controlled matrices, and erodible/degradable matrices.

For oral administration in the form of a tablet or capsule, the active drug component can be combined with a non-toxic, pharmaceutically acceptable excipients such as binding agents (*e.g.*, pregelatinized maize starch, polyvinylpyrrolidone or hydroxypropyl methylcellulose); fillers (*e.g*., lactose, sucrose, glucose, mannitol, sorbitol and other reducing and non-reducing sugars, microcrystalline cellulose, calcium sulfate, or calcium hydrogen phosphate); lubricants (*e.g.*, magnesium stearate, talc, or silica, steric acid, sodium stearyl fumarate, glyceryl behenate, calcium stearate, and the like); disintegrants (*e.g.*, potato starch or sodium starch glycolate); or wetting agents (*e.g.*, sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like. For oral administration in liquid form, the drug components can be combined with non-toxic, pharmaceutically acceptable inert carriers (*e.g.*, ethanol, glycerol, water), suspending agents (*e.g.*, sorbitol syrup, cellulose derivatives or hydrogenated edible fats), emulsifying agents (*e.g.*, lecithin or acacia), non-aqueous vehicles (*e.g.*, almond oil, oily esters, ethyl alcohol or fractionated vegetable oils), preservatives (*e.g.*, methyl or propyl-p-hydroxybenzoates or sorbic acid), and the like. Stabilizing agents such as antioxidants (BHA, BHT, propyl gallate, sodium ascorbate, citric acid) can also be added to stabilize the dosage forms.

The tablets can be coated by methods well known in the art. The compositions of the invention can be also introduced in microspheres or microcapsules, *e.g.*, fabricated from polyglycolic acid/lactic acid (PGLA) (*see*, *e.g*., U.S. Patents No. 5,814,344; 5,100,669 and 4,δ49,222; PCT Publications No. WO95/11010 and WO93/07861). Liquid preparations for oral administration can take the form of, for example, solutions, syrups, emulsions or suspensions, or they can be presented as a dry product for reconstitution with water or other suitable vehicle before use. Preparations for oral administration can be suitably formulated to give controlled or postponed release of the active compound. A particular example of an oral time-controlled release pharmaceutical formulation is described in U.S. Patent No. 5,366,738.

The active drugs can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines, as is well known.

Drugs of the invention may also be delivered by the use of monoclonal antibodies as individual carriers to which the compound molecules are coupled. Active drugs may also be coupled with soluble polymers as targetable drug carriers. Such polymers can include polyvinylpyrrolidone, pyran copolymer, polyhydroxy-propyl methacrylamide-phenol, polyhydroxy-ethyl-aspartamide-phenol, or polyethyleneoxide-polylysine substituted with palmitoyl residues. Furthermore, active drug may be coupled to a class of biodegradable polymers useful in achieving controlled release of a drug, for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

For administration by inhalation, the therapeutics according to the present invention can be conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered amount. Capsules and cartridges of, *e.g.*, gelatin for use in an inhaler or insufflator can be formulated containing a powder mix of the compound and a suitable powder base such as lactose or starch.

The formulations of the invention can be delivered parenterally, *i.e*., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, *e.g.*, in ampoules or in multi-dose containers, with an added preservative. The compositions can take such forms as excipients, suspensions, solutions, or emulsions in oily or aqueous vehicles, and can contain formulatory agents such as suspending, stabilizing and/or dispersing agents. Alternatively, the active ingredient can be in powder form for reconstitution with a suitable vehicle, *e.g.*, sterile pyrogen-free water, before use.

Compositions of the present invention can also be formulated for rectal administration, *e.g.*, as suppositories or retention enemas (*e.g.*, containing conventional suppository bases such as cocoa butter or other glycerides).

As disclosed herein, an 1-aminocyclohexane derivative can be mixed with excipients which are pharmaceutically acceptable and compatible with the active ingredients. In addition, if desired, the preparations may also include minor amounts of auxiliary substances such as wetting or emulsifying agents, pH buffering agents, and/or agents that enhance the effectiveness of the pharmaceutical composition. These auxiliary molecules can be delivered systemically or locally as proteins or by expression of a vector that codes for expression of the molecule. The techniques described above for the delivery of 1-aminocyclohexane derivatives can also be employed for the delivery of auxiliary molecules.

Although the active agents of the present invention may be administered in divided doses, for example, two or three times daily, a single daily dose of the 1-aminocyclohexane derivative is preferred.

Preferred specific amounts of the 1-aminocyclohexane derivative which may be used in unit dosage amounts of the invention include, for example, 5mg, 10 mg, 15 mg, and 20 mg for memantine and 5 mg, 10 mg, 20 mg, 30 mg, and 40 mg for neramexane.

According to a specific embodiment, a controlled release formulation (also herein after referred to as a "controlled release composition") of the 1-aminocyclohexane derivative is utilized in order to provide an enhanced effect that cannot be achieved by conventional immediate release dosing. The use of a controlled release form may be specially useful for providing a constant level of the 1-aminocyclohexane derivative in order to avoid dosage peaks and valleys in those mammals who have meals at irregular times or those who frequently eat snacks between meals.

Controlled release formulations have been described in U.S. Pat. No. 4,615,698 which have been based on an osmotic dosage form which is designed to collapse and cause the faced surfaces to come into a closed contacting arrangement as the drug is delivered through a passageway in the semi-permeable wall of the dosage form. In addition, U.S. Pat. No. 4,503,030 discloses an osmotic dosage form which has a passageway and a semi-permeable membrane consisting of a particular cellulose polymer and a pH sensitive material which could be an enteric coating material. This patent describes the use of 1:1 mixtures of a pH sensitive material and cellulose polymer which are applied at a level of about 7% by weight based on the total weight of the osmotic core tablet and coating material.

The invention also provides a pharmaceutical pack or kit comprising one or more containers containing one or more of the ingredients of the formulations of the invention. In a related embodiment, the present invention provides a kit for the preparation of the pharmaceutical compositions of the invention, said kit comprising an 1-aminocyclohexane derivative in a first container, and, optionally, instructions for admixing the 1-aminocyclohexane derivative and/or for administration of the compositions. Each container of the kit may also optionally include one or more physiologically acceptable carriers and/or excipients and/or auxiliary substances. Associated with such container(s) can be a notice in the form prescribed by a governmental agency regulating the manufacture, use or sale of pharmaceuticals or biological products, which notice reflects approval by the agency of manufacture, use or sale for human administration.

The compositions may, if desired, be presented in a pack or dispenser device which may contain one or more unit dosage forms containing the active ingredient. The pack may, for example, comprise metal or plastic foil, such as a blister pack. The pack or dispenser device may be accompanied by instructions for administration. Compositions of the invention formulated in a compatible pharmaceutical carrier may also be prepared, placed in an appropriate container, and labeled for treatment of an indicated condition.

### Effective Dose and Safety Evaluations

According to the methods of the present invention, the pharmaceutical compositions described herein are administered to a patient at therapeutically effective doses, preferably, with minimal toxicity. The Section entitled "Definitions" provides definitions for the terms "neurologically effective dose" and "therapeutically effective dose".

The efficacy of the 1-aminocyclohexane derivatives of the invention can be determined using *in vitro* assays in cultured cells, which are known to secrete into the condition APP peptides. Suitable cell lines include human and animal cell lines, such as human neuroblastoma cell lines (*e.g.*, SK-N-SH [ATCC HTB-11], SK-N-MC [ATCC HTB-10], IMR-32 [ATCC CCL-127], MC-IXC [ATCC CRL-2270]), human neuroglioma cell lines, human HeLa cells, human kidney cell line HEK-293, primary human endothelial cells (*e.g.*, HUVEC cells), primary human fibroblasts or lymphoblasts, primary human mixed brain cells (including neurons, astrocytes, and neuroglia), Chinese hamster ovary (CHO) cells, and the like. Preferred for use according to the present invention are human cell lines that express APP variants or that overproduce Aβ, *e.g.*, APP variants having one or several amino acid substitutions directly at the N-terminus of the Aβ cleavage site (*e.g.*, K293 cells which express an APP DNA bearing a double mutation [Lys⁵⁹⁵ → Asn⁵⁹⁵ and Met⁵⁹⁶→ Leu⁵⁹⁶] found in a Swedish FAD family, which produce approximately six-to-eight-fold more Aβ than cells expressing normal APP, as disclosed in the U.S. Patent No. 6,284,221). The levels of these secreted derivatives of APP (*i.e.*, sAPPα, total Aβ, Aβ₄₀, or Aβ₄₂) can be estimated, for example, by immunodetection (*e.g.*, Western blotting or immunoprecipitation) using various specific polyclonal and monoclonal antibodies.

Additionally, the efficacy of the 1-aminocyclohexane derivatives of the invention can be determined using such *in vitro* pharmacological tests as measurements of displacement of [³H]MK-801 binding in rat or human brain tissue, blocking of NMDA receptor channels in cultured neurones and heterologous expression systems, anticonvulsive effects *in vivo*, correlation between channel-blocking and anticonvulsive action, protection against cerebral ischemia, protection against NMDA-induced mortality, etc. (*see, e.g.*, U.S. Patent No. 5,061,703).

Following methodologies which are well-established in the art, effective doses and toxicity of the compounds and compositions of the instant invention, which performed well in *in vitro* tests, are then determined in preclinical studies using small animal models (*e.g.*, mice or rats) in which the 1-aminocyclohexane derivatives has been found to be therapeutically effective and in which these drugs can be administered by the same route proposed for the human clinical trials. Preferred animal models of the invention are transgenic models of AD disclosed in Example 2, *infra*.

For any pharmaceutical composition used in the methods of the invention, the therapeutically effective dose can be estimated initially from animal models to achieve a circulating plasma concentration range that includes the IC₅₀ (*i.e.*, the concentration of the test compound which achieves a half-maximal inhibition of NMDA receptor activity in the relevant areas of the brain). Dose-response curves derived from animal systems are then used to determine testing doses for the initial clinical studies in humans. In safety determinations for each composition, the dose and frequency of administration should meet or exceed those anticipated for use in the clinical trial.

As disclosed herein, the dose of the components in the compositions of the present invention is determined to ensure that the dose administered continuously or intermittently will not exceed an amount determined after consideration of the results in test animals and the individual conditions of a patient. A specific dose naturally varies depending on the dosage procedure, the conditions of a patient or a subject animal such as age, body weight, sex, sensitivity, feed, dosage period, drugs used in combination, seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques. As disclosed herein, an appropriate dose of an 1-aminocyclohexane derivative is generally in the range of 0.016-1.66 mg per kg of body weight.

Toxicity and therapeutic efficacy of the compositions of the invention can be determined by standard pharmaceutical procedures in experimental animals, *e.g.*, by determining the LD₅₀ (the dose lethal to 50% of the population) and the ED₅₀ (the dose therapeutically effective in 50% of the population). The dose ratio between therapeutic and toxic effects is the therapeutic index and it can be expressed as the ratio ED₅₀/LD₅₀. Compositions that exhibit large therapeutic indices are preferred.

The data obtained from animal studies can be used in formulating a range of doses for use in humans. The therapeutically effective doses of 1-aminocyclohexane derivatives in humans lay preferably within a range of circulating concentrations that include the ED₅₀ with little or no toxicity. For example, such therapeutically effective circulating concentration for memantine is approximately 1 µM (see, *e*.*g.*, Kornhuber and Quack,. Neurosci Lett. 195(2):137-139,1995). The dosage can vary within this range depending upon the dosage form employed and the route of administration utilized. Ideally, a single dose of each drug should be used daily.

The drug of the invention is not only highly effective at relatively low doses but also possesses low toxicity and produces few side effects. Indeed, the most common side effect resulting from the use of 1-aminocyclohexane derivatives of the invention is a minor motor and cognitive impairment (reflected, *e.g.*, in nausea, vomiting, dizziness, or confusion).

### EXAMPLES

The following Examples illustrate the invention without limiting its scope.

### EXAMPLE 1: Determination of the Effect of Therapeutic Concentrations of Memantine on the Levels of Secreted APP Derivatives in Human Neuroblastoma Cells

### Background

Memantine is a moderate affinity, uncompetitive (open channel) NMDA receptor antagonist. It exhibits strong voltage-dependent channel blocking characteristics and fast channel blocking/unblocking kinetics. Memantine has been shown to provide neuroprotection and improve learning and memory in several animal models. Clinically, memantine reduces the decline of cognitive function in moderate to severe Alzheimer's disease (AD) patients. (for review see Lahiri et al., Curr. Drug Targets 2003, 4(2): 97-112). Brains of subjects suffering from amyloidopathies such as Alzheimer's Disease (AD), Down's Syndrome, or HCHWA-D, are characterised by the presence of extracellular aggregates of Aβ peptides which are deposited as amyloid fibrils or amorphous aggregates and are thought to play a crucial role in desease pathogenesis. Two major highly fibrillogenic forms of amyloid beta peptides are the short form that ends at the 40th residue (Aβ₄₀) and the long form that ends at the 42nd residue (Aβ₄₂). The extracellular deposition of these highly fibrillogenic Aβ peptides occurs due to aberrant processing of the full-length beta-amyloid precursor protein (APP) by various proteolytic enzymes known as secretases. In this context, it is important to identify drugs which are able to affect the levels of amyloidogenic and potentially toxic Aβ peptides.

The processing of APP can be detected in cell cultures. For example, neuroblastoma cells are known to secrete APP derivatives, which are shorter in length than the full length intracellular APP, into the conditioned medium. The levels of these secreted derivatives of APP can be estimated by probing the conditioned media with specific antibodies to APP using, *e.g.*, the method of Western blotting or ELISA (enzyme linked immunosorbent assay).

In the present Example, the inventors investigated the effect of therapeutic concentrations of memantine (1-4 µM) on the levels of secreted amyloid peptides, sAPPα, Aβ₄₀, and Aβ₄₂ in the conditioned medium of human neuroblastoma (SK-N-SH) cells. SK-N-SH cells were treated with 1-4 µM memantine for up to 12 days and the levels of sAPP and Aβ₄₀ in the conditioned media were measured by Western immunoblotting and ELISA assays, respectively. Memantine (2-4 µM for 6-12 days) significantly decreased sAPP levels in the conditioned media. A lower concentration of memantine (1 µM for 6-12 days) exhibited a trend towards a decrease in the levels of sAPP. Determination of Aβ₄₀ levels also indicated a decrease in its levels. Cell viability and toxicity were not affected by memantine at the tested concentrations. These data indicate that memantine, at therapeutic concentrations, affects APP processing and may potentially inhibit the accumulation of fibrillogenic Aβ peptides.

### Experimental design

*Cell culture and drug treatment.* Human neuroblastoma (SK-N-SH, ATCC HTB-11, Biedler et al., Cancer Res. 1973, 33(11): 2643-52) cells were seeded at approximately 2x10⁶ cells/well in a 6-well plate. Thereafter, drug concentrations of memantine were initiated at 0 µg/ml (control), 0.25 µg/ml (1 µM), 0.5 µg/ml (2 µM), and 1.0 µg/ml (4 µM) in MEM media supplemented with 1% FBS and 1x antibiotic. Treatment period was 12 days with a collection of conditioned media (CM) at 3 day intervals. Following collection, fresh memantine-media was added and the process repeated until harvest on day 12. The levels of both sAPP and Aβ₄₀ were determined as described below.

*Analysis of sAPP levels by Western blotting.* Levels of total sAPP in the CM samples were measured by denaturing polyacrylamide gel electrophoresis (SDS-PAGE) followed by the Western immunoblotting using mAb22C11 antibody. Samples were loaded on the gel at equal volume of conditioned media (30µg protein/sample). The density of specific APP bands in the blot was quantified using the NIH Image software. Levels of APP were expressed as a percent of controls. Levels of the constitutively expressed β-actin protein were measured in parallel as an internal control.

*Analysis of Aβ levels by ELISA.* Quantitative solid phase sandwich ELISA (enzyme linked immunosorbent assay) was used to measure Aβ₄₀ levels in the conditioned media samples. Specifically, an affinity purified anti-human Aβ (35-40) rabbit IgG was used as a capture antibody, and affinity purified HRP-conjugated anti-human Aβ (11-28) rabbit IgG Fab was used as a detection antibody (both reagents from IBL (Japan)). Since an antibody against Aβ (11-28) was used as a secondary conjugated antibody, human Aβ₄₀ variants which cleaved at N-terminus sites were also detectable in the ELISA assay. TMB was used as a coloring agent (Chromogen). The measurement range was from 15.6 to ∼1,000 pg/ml (3.6 to ∼230.9 pmol/l).

*Cell Toxicity and Viability Assays.* Cell viability in control and memantine-treated samples was measured using MTT (the tetrazolium dye 3-[4,5-dimethylthiazol-2-yl]-2,5-diphenyltetrazolium bromide). Cellular toxicity was measured uding lactate dehydrogenase (LDH) (Sigma, St. Louis, MO) assay.

*Data Analysis.* Data were analyzed from 3-6 independent cell culture experiments. Statistical analysis (*e.g.*, analysis of variance and post-hoc tests for multiple comparisons) was performed using the SPSS program (Statistical Products and Services Solutions, Chicago, IL) and included the mean and the standard error of the mean.

### Results

Human neuroblastoma (SK-N-SH) cells were grown in the presence of 0 µg/ml (control), 0.25 µg/ml (1 µM), 0.5 µg/ml (2 µM), and 1.0 µg/ml (4 µM) of memantine for 12 days. Samples of conditioned media were collected at 3 day intervals and the levels of both sAPP and Aβ₄₀ were determined by Western blotting and ELISA, respectively. Although no significant changes in the levels of sAPP were observed on day 3 (Figure 1), a dose-dependent decrease in sAPP levels was observed on day 6 (Figure 2), persisting or further decreasing when measured on day 9 and day 12 (Figure 3). Like sAPP, no significant changes in levels of Aβ₄₀ were observed on day 3 (Figure 4). Notably, there was a significant decrease (compared to negative control) in Aβ₄₀ levels on day 6 (Figure 5) and day 9 (Figure 6).

The effect of memantine on cellular viability and toxicity was also determined. Cell viability was assessed using a colorimetric MTT assay. As shown in Figure 7, an increase in cell viability was observed in the memantine-treated vs. untreated (control) plates making the significance of the decrease in sAPP and Aβ₄₀ levels even more prominent (as normalized to cell viability). The LDH assay of toxicity revealed no toxicity towards the cells at any memantine dosage used (Figure 8).

Taken together, a gradual decrease in both sAPP and Aβ₄₀ levels in conditioned media of human neuroblastoma (SK-N-SH) cells was observed in the presence of therapeutic non-toxic (1-4 µM) doses of memantine.

The present inventors are using the same experimental approach to determine the effect of therapeutic concentrations of memantine on the level of Aβ₄₂. By using various unrelated uncompetitive and competitive antagonists of NMDA receptors, and antagonists of non-NMDA receptors (*e.g.*, AMPA receptors), the present inventors are also determining whether memantine decreases the levels of sAPPα, Aβ₄₀ and Aβ₄₂ in the conditioned media via its activity on NMDA receptors.

### Conclusions

The present data indicate that the treatment of human neuroblastoma cells SK-N-SH with theraputic doses of memantine (1-4 µM) results in a decrease in sAPP and Aβ₄₀ levels in the conditioned media. Cell viability and toxicity, as determined by MTT and LDH assays, respectively, are not affected by memantine at the above concentrations. The observed decrease in sAPP and Aβ₄₀ levels at therapeutic concentrations of memantine suggests that memantine may decrease the deposition of fibrillogenic Aβ peptides in the brain.

### EXAMPLE 2: Determination of the Effects of Administering Therapeutic Doses of Memantine and Other 1-aminocyclohexane Derivatives on the Levels of Aβ₄₀ and Aβ₄₂ in the Brains of Mouse Models of Alzheimer's Disease

AD appears to have a heterogeneous etiology with a large percentage termed sporadic AD arising from unknown causes and a smaller fraction of early onset familial AD (FAD) caused by mutations in one of several genes, such as the beta-amyloid precursor protein (APP) and presenilins (PS1, PS2). These proteins along with *tau*, secretases, such as β-amyloid cleaving enzyme (BACE), and apolipoprotein E play important roles in the pathology of AD (for recent review see Lahiri et al., Curr. Drug Targets, 4:97-112, 2003).

In some individuals with early-onset AD, the illness may be inherited as an autosomal dominant (*i.e.*, only a single copy of the mutant gene is necessary to cause the disease). Such mutations are identified in at least three different genes: APP, presenilin 1 (PS1) and presenilin 2 (PS2) (Price et al., Annu. Rev. Genet., 1998, 32: 461-493; Hardy et al., Science, 1998, 282: 1075-1079; Tanzi, Neuron, 2001, 32: 181-184; Selkoe, *ibid.*, pp. 177-180; Sherrington et al., Nature, 1995, 375: 754-760; Levy-Lahad et al., Science, 1995, 269: 973-977; Rogaev et al., Nature, 1995, 376: 775-778).

A variety of APP mutations reported in cases of FAD (familial AD) are near cleavage sites involved in formation of Aβ (*see, e.g.*, Goate et al.,1991, Nature, 349:704-706; Harlan et al., 1991, Nature, 353:844-846; Murrell et al., 1991, Science, 254:97-99; and Mullan et al., 1992, Nature Genet., 1:345-347). The APP 717 mutation is located near the C-terminus of Aβ and facilitates β-secretase activity, leading to increased secretion of the longer and more toxic Aβ peptide, Aβ₄₂. This longer Aβ₄₂ peptide is thought to promote the formation of Aβ aggregates and amyloid plaques. The APPswe mutation, a double mutation at the N-terminus of Aβ, enhances BACE1 cleavage and is associated with elevated levels of Aβ peptides, including Aβ₄₂. In contrast, APP mutations within the Aβ peptide domain (for example, APP-E693Q, A692G or E693G) do not elevate the level of Aβ but may cause amyloidosis by increasing Aβ oligomer or protofibril formation.

PS1 and PS2 encode highly homologous 43- to 50-kD multipass transmembrane proteins that are processed to stable N-terminal and C-terminal fragments, and are widely expressed but at low abundance in the central nervous system. PS1 influences APP processing (Borchelt et al. Neuron, 1997, 19: 939-945; Wong *et al.,* 2002, *supra*). The PS1 gene has been reported to harbor more than 80 different FAD mutations (*see* AD mutation database, http://molgen-www.uia.ac.be), whereas only a small number of mutations have been found in PS2-linked families. The vast majority of abnormalities in PS genes are missense mutations that result in single amino acid substitutions, which in general seem to influence secretase activity and increase the generation of the Aβ₄₂ peptide.

The concentrations of 1-aminocyclohexane derivative (*e.g*., memantine or neramexane) resulting in therapeutically meaningful decrease in the processing and/or secretion of the amyloidogenic Aβ in cell cultures are further tested *in vivo* by monitoring of Aβ levels in transgenic animal models of AD, such as the mouse animal models expressing APP minigenes that encode FAD-linked APP mutants (*e.g.*, swe or 717, as disclosed, *e.g.*, in U.S. Patent No. 5,912,410) or the double mutant mouse model descibed by Borchelt et al. (Neuron, 19: 939-945, 1997). The latter transgenic mice coexpress an early-onset familial AD (FAD)-linked human presenilin 1 (PS1) variant (A246E) and a chimeric mouse/human APP harboring mutations linked to Swedish FAD kindreds (APPswe). These mice develop numerous amyloid deposits much earlier than age-matched mice expressing APPswe and wild-type human PS1. Expression of APP minigenes that encode FAD-linked APP mutants and, in particular, co-expression of the mutant human PS 1 A246E and APPswe elevates levels of Aβ in the brain, and these mice develop numerous diffuse Aβ deposits and plaques in the hippocampus and cortex (Calhoun et al., Proc. Natl. Acad. Sci. USA, 1999, 96: 14088-14093). Similarly to humans suffering from AD, these and other transgenic animal models are characterized by various cognitive defects such as loss of neurons, learning deficits, problems in object recognition memory, and problems with alternation-spatial reference and working memory (Chen et al., Nature, 2000, 408: 975-979).

Specifically, two groups of transgenic animals are being studied: a control group, which receives no treatment, and an experimental group, which receives the 1-aminocyclohexane derivative (such as memantine or neramexane). Drug administration is carried on over defined periods of time and is followed by testing (*e.g.*, using immunodetection and histochemistry) (i) the level of various APP peptides (*e.g.*, sAPPα, Aβ₄₀ or Aβ₄₂) in the body fluids and (ii) the amount of β-amyloid plaques within the brain. The decrease observed in the experimental group (as compared to the control group) is used as a measure of the effectiveness of the 1-aminocyclohexane derivative therapy of the invention. The transgenic animal models are further used to determine the optimal dosages, efficacy, toxicity as well as side effects associated with the 1-aminocyclohexane derivative therapy of the invention.

Based on the cell culture data on APP processing, it is expected that memantine will decrease the deposition of Aβ₄₀ and Aβ₄₂ in this transgenic mouse model of AD.

### EXAMPLE 3: Determination of the Effects of Administering Therapeutic Doses of Memantine and on spatial learning in a transgenic mouse model of Alzheitmer's disease

### Background

In the mammalian brain, NMDA receptors are involved in important physiological functions such as synaptic plasticity and synapse formation, which play important roles in memory, learning and the formation of neural networks during development (Mayer and Westbrook, 1987). Given the critical role of NMDA receptors in learning and memory (Morris, 1989; Tsien et al., 1996), it may appear counter-intuitive that an NMDA receptor antagonist could improve the symptomatology of Alzheimer's disease (AD). Several NMDA receptor antagonists possessing high affinity for NMDA receptors [e.g., (+) MK-801] have been found to cause neurobehavioral adverse effects such as hallucination and cognitive impairment (Benvenga and Spaulding, 1988; Abi-Saab et al., 1998). These adverse events have largely limited the clinical development of high affinity NMDA receptor antagonists. An alternative approach to avoid such side effects is to produce a partial rather than complete blockade of the NMDA receptor. Partial receptor blockade can be achieved, for example, by low affinity NMDA receptor antagonists, which typically possess a better therapeutic window than high affinity NMDA receptor antagonists (Rogawski, 2000). Memantine, a low to moderate affinity NMDA receptor antagonist, has been shown to improve performance in several pharmacological models of impaired learning and memory (Zajaczkowski et al.,1996; Wenk et al., 1997), in aged rats with impaired baseline memory function (Barnes et al., 1996) and in patients with moderate to severe AD (Reisberg et al., 2003; Tariot et al., 2004).

One of the most distinct pathological hallmarks of AD is extracellular deposition of β-amyloid (Aβ) plaques in select brain regions. A subset of AD cases exhibit early onset and are familial (FAD). FAD is caused by mutations in the presenilin 1 (PS 1), presenilin 2 (PS 2) or amyloid precursor protein (APP) genes. Such mutations lead to enhanced production of highly fibrillogenic Aβ1-42 peptides (Borchelt et al., 1997; Holcomb et al., 1998). Several lines of evidence suggest that Aβ toxicity may be related to elevated levels of glutamate and/or overactivity of NMDA receptors. For example, APP is expressed by glutamatergic neurons (Ouimet et al., 1994), and the cellular damage in the brains of AD patients is found predominantly in areas that display glutamatergic synaptic plasticity (Arendt et al., 1998). Infusion of Aβ in rat brains produces deficits in learning and memory (Sweeney et al., 1997) and impairment in long-term potentiation (LTP), a model of activity-dependent synaptic plasticity that may underlie some forms of learning and memory (Stephan et al., 2001; Walsh et al., 2002). Transgenic mice overexpressing Aβ and APP also exhibit age-dependent cognitive decline (Chapman et al., 1999; Puoliväli et al., 2002), and glutamate is known to exacerbate Aβ-induced impairment of LTP (Nakagami and Oda, 2002). Moreover, in a recent study, memantine protected rat hippocampal cells from Aβ-induced apoptosis (Miguel-Hidalgo et al., 2002). Even in the absence of either Aβ or APP, over activation of NMDA receptors can decrease synaptic plasticity and learning. For example, the generation of LTP can be impaired by a high concentration of NMDA (Katagiri et al., 2001), and systemic administration of a non-convulsive dose of NMDA has been shown to impair passive avoidance learning in rats (Zajaczkowski et al., 1997).

The finding that down-regulation of the glial glutamate transporter, GLT-1 (EAAT-2) occurs in AD patients also supports the idea that synaptic levels of glutamate and therefore NMDA receptor activity may increase in AD (Masliah et al., 1996). Interestingly, mice lacking GLT-1 also show elevated synaptic levels of glutamate and impaired hippocampal LTP, which are partially restored to normal levels by a low dose of NMDA receptor antagonist (Katagiri et al., 2001), and APP transgenic mice show impaired glial glutamate transporter activity (Masliah et al., 2000). Collectively, these findings suggest that the over activation of NMDA receptors and/or elevated levels of glutamate in the synapse can exacerbate the neurotoxic and memory-impairing effects of Aβ and APP.

In the present study, the effect of sub-chronic oral administration of memantine on hippocampus-based spatial learning and other general behaviors was determined in mice carrying mutated human APP(swe) and PS1 (A246E) genes. These mice develop age-dependent memory impairment and exhibit age-related increases in Aβ levels in several brain regions (Liu et al., 2002; Puoliväli et al., 2002).

### Experimental design

Transgenic mice expressing either human PS1 harboring the familial AD-linked A246E mutation or chimeric mouse/human APP695 harboring a human Aβ domain and mutations (K595N, M596L) linked to Swedish familial AD pedigrees (APPswe) (Borchelt et al., 1997) were back-crossed to C57BL/6J for 16 generations and then crossed together to generate double transgenic mice co-expressing both transgenes. In all tests, 8-month-old double-mutant male mice (APP/PS1; n=45) and their non-transgenic littermates (NT; n=36,) were used. At this age, APP/PS 1 mice exhibit increased brain levels of β-amyloid peptides (Wang et al., 2003). The therapeutic dose of memantine was defined as the dose producing a steady-state plasma drug level of ∼1 µM and was determined in 8-month-old male C57 BL/6J mice (background strain of the transgenic mice).

Throughout the experiment animals were housed individually in a controlled environment (temperature 21 ± 1°C, humidity 50 ± 10%, light period 07:00-19:00 h). Food and water were available ad libitum. The experiments were conducted according to the Council of Europe (Directive 86/609) and Finnish guidelines, and approved by the State Provincial Office of Eastern Finland.

*Dose-finding pilot study*: A pilot study was undertaken to determine the therapeutic dose of memantine [i.e., the dose of memantine producing a steady-state plasma drug level of around 1µM, which several preclinical and clinical studies have indicated is therapeutic (Kornhuber and Quack, 1995; Zajaczkowski et al., 1996)] to be used in subsequent experiments in transgenic mice. Memantine (Forest Research Institute, Jersey City, NJ) was administered orally (via drinking water) to male C57BL/6J mice at the doses of 10 mg/kg/day (n = 10), 30 mg/kg/day (n = 10) and 100 mg/kg/day (n = 10) for 4 weeks. The placebo group (n = 10) had drinking water without memantine. Blood samples were taken from the femoral vein 4 weeks after the initiation of drug treatment to determine the steady-state plasma concentration of memantine. Plasma samples were analyzed at Merz Pharmaceuticals GmbH (Frankfurt am Main, Germany) using a gas chromatograph system coupled with a mass selective detector (Kornhuber and Quack., 1995).

*Memantine treatment*: Based on the pilot study, the dose of 30 mg/kg/day (see Results section for details) was chosen for the behavioral study and administered in drinking water for 3 weeks. Memantine was administered to 23 APP/PS 1 mice and 19 NT mice. The placebo group (APP/PS1: n = 22; NT: n = 17) received drinking water without memantine. Behavioral testing started 2 weeks after treatment onset and continued for one week. After two weeks of treatment, mice were tested for exploratory activity, isolation-induced aggression, and performance in the Morris water maze.

*Exploratory activity*: TruScan® (Coulbourn Instruments, CO, USA) automated activity monitor based on infrared photo detection was used for monitoring exploratory activity. The system consists of a transparent observation cage (26x26x39 cm) and two rings of photo detectors enabling separate monitoring of horizontal (XY-movement over time) and vertical activity (rearing). Activity was measured for 10 min in two separate sessions separated by 48 h.

*Isolation-induced aggression*: All test mice ('residents') had been housed in individual cages for at least 3 weeks prior to the start of this test. 'Intruders' were NT male C57B1/J6 mice, 16-20 weeks old at the time of testing, housed in groups of 4-8 since weaning. A randomly chosen intruder was placed in the resident's cage, and aggression of the resident was assessed by measuring attack latency, i.e. the time in seconds between the introduction of the intruder into the cage and the first attack by the resident. The experimenter was blind to genotype and drug treatment.

*Morris water maze*: The Morris water maze was used to measure spatial learning and memory. The apparatus was a black plastic pool with a diameter of 120 cm. A black escape platform (square, 14 x 14 cm) was located 1.0 cm below (hidden) the water surface. The temperature of the water was kept constant throughout the experiment (20 ± 0.5°C), and a 10-min recovery period was allowed between the training trials. First, the mice were pre-trained to find and climb onto the platform for two days by using an alley (1 m x 14 cm x 25 cm) leading to the platform located 1 cm below the water. The training consisted of 8 consecutive days of testing, with 5 trials per day. If the mouse failed to find the escape platform within the maximum time (60 seconds), the animal was placed on the platform for 10 s by the experimenter. During the first 5 days of testing the mice were trained with a hidden platform. The platform location was kept constant and the starting position varied between four constant locations at the pool rim. Mice were placed in the water with their nose pointing towards the wall at one of the starting points in a random manner. On the sixth day, the platform was removed and the mice were allowed to swim for 60 s to determine their search bias. On testing days 7 and 8, a black curtain was hung around the swimming pool in order to conceal all extra-maze visual cues. The mice were trained to find a visible platform, which had a 10 cm high pole with a white flag and which was changed every trial to a new position. Timing of the latency to find the submerged platform was started and ended by the experimenter. A computer connected to an image analyzer (HVS Image®, Hampton, UK) monitored the swim pattern. During the water maze training, we measured swimming speed and latency to find the platform. The wall-swimming tendency (thigmotaxis) was assessed by dividing the pool into 3 concentric zones of equal surface area and calculating the time spent in the outer zone. Search bias during the probe trial was measured by calculating the time the mice spent in the vicinity of where the platform was previously located. We defined this as a target area centered on the platform with a diameter of 30 cm. This target area comprised 6.25 % of the total surface area, thus a random swim for 60 s in the pool would yield a dwell time of 3.75 s in the target area during the probe trial.

*Statistical analysis* All statistical analyses were performed using SPSS for Windows software, version 11.5.1 (SPSS, Chicago, IL). The effects of genotype, treatment, training day, and their interaction with the behavioral parameters of exploratory activity and performance in the Morris water maze were evaluated by analysis of variance (ANOVA) for repeated measures. Attack latency from the isolation-aggression test was analyzed by two-way ANOVA with genotype and treatment as factors.

### RESULTS

*Memantine plasma concentrations*: The steady-state plasma levels following oral administration of 10, 30 and 100 mg/kg/day Memantine were 0.49 ± 0.06, 1.14 ± 0.07 and 5.54 ± 0.40 µM (mean ± SEM), respectively. Based on these data, the dose of 30 mg/kg/day, which produces the therapeutic steady-state plasma level of around 1µM, was chosen for all behavioral studies.

*Exploratory activity*: APP/PS1 and NT mice were first tested in an automated activity monitor to detect genotype and drug effects on motor and exploratory activity. ANOVA revealed a significant genotype effect. The APP/PS1 mice exhibited less horizontal activity (Fig 9, F(1, 77) = 13.0, p = 0.001) and less rearing (Fig 9, F(1, 77) = 35.0, p < 0.001) than NT mice. Memantine did not significantly affect either measure of exploratory activity.

*Isolation-induced aggression*: When confronted with an intruder mouse, APP/PS1 mice exhibited a shorter latency to attack the intruder than N'T controls (Fig. 10; F(1, 56) = 3.9, p = 0.05). The increased aggressive behavior observed in APP/PS1 mice was not significantly modified by memantine (Fig. 10).

*Morris water maze*: The overall ANOVA revealed both a genotype (F(1, 77) = 12.5, p = 0.001) and a drug effect (F(1, 77) = 8.0, p = 0.006) in spatial learning. Placebo-treated APP/PS1 mice were slower than NT controls in finding the hidden platform (Fig. 11A; F(1,40) = 8.9, p = 0.005). Treatment with memantine reduced the escape latency in APP/PS1 mice compared to placebo- treated APP/PS1 mice (Fig. 11B; F(1,43) = 6.0, p = 0.02). In fact, the performance level of memantine-treated APP/PS1 mice did not differ from that of placebo-treated NT mice (one-way ANOVA with four groups, followed by Tukey's post-hoc test, p = 0.96). There was also a trend towards improved performance in NT mice treated with memantine; however, this effect was not significant (Fig. 11C; F(1,34) = 3.0, p = 0.09). APP/PS1 mice were also slower than their N'T littermates in finding the visible platform (Fig. 11A; F(1, 77) = 15.8, p < 0.001). However, memantine did not show a significant improvement in this paradigm (Fig. 11B). Swimming speed was not affected by genotype (F(1, 77) = 0.27, p > 0.6) or drug treatment (F(1, 77) = 0.94, p > 0.3).

Initially, the natural tendency of mice is to remain close to the pool wall to find an escape from the water. However, they soon realize there is no escape through the wall and begin to search for the platform in the middle of the pool. To further analyze search pattern, the time spent in the outer zone of the pool was separately measured. The total time spent in the outer zone for APP/PS1 mice was significantly greater than for NT mice (Fig. 11D; F(1, 77) =18.3, p < 0.001). Memantine significantly reduced the time spent in the outer zone (F(1, 77) = 11.7, p = 0.001), and this effect was significant for both APP/PS1 mice (Fig. 11E; F(1,43) = 4.7, p = 0.04) and NT mice (Fig. 11F; F(1,34) = 9.8, p = 0.004).

The strength of the learned spatial search bias was assessed during a probe trial on the sixth day without the platform. Mice in all groups spent more time in the vicinity of the platform location than would be expected by random swimming (3.75 s out of 60 s; see Methods). The time spent in the target area for the different groups was as follows: NT (placebo): 36.0 ± 2.0 s (mean:L sem), NT (memantine): 36.1 ± 2.6 s, APP/PS1 (placebo): 35.5 ± 2.3 s, APP/PS1 (memantine): 39.0 ± 2.0 s. Group differences were not significant.

### CONCLUSIONS

Following 3-4 weeks of oral administration at therapeutic plasma concentrations, memantine significantly improved the learning phase of spatial navigation in APP/PS1 mice, which exhibit age-dependent impairment in spatial learning (Fig. 11B). Memantine did not affect spontaneous locomotor activity or special motor patterns such as swimming in the water maze.

No changes in spontaneous rearing were observed or horizontal locomotion in either APP/PS1 or NT mice treated with memantine. Some of the characteristic effects of (+)MK-801 in rodents are dose-dependent hyperactivity (French et al., 1991; Hargreaves and Cain, 1995), impairment in water maze performance and increased wall-clinging (thigmotaxis) (Cain et al., 1996). In contrast, memantine treatment improved water maze learning in APP/PS1 mice and reduced thigmotaxis. These effects of memantine are in agreement with the high tolerability profile of memantine observed in clinical trials.

Memantine treatment resulted in a significant improvement in water maze acquisition in APP/PS1 mice. In an earlier study in Fisher 344 rats, improved water maze learning with memantine treatment of 30 mg/kglday for 8 weeks had been reported (Barnes et al., 1996). However, in that study, the effect of memantine was apparent at the later stages of water maze learning by improved search bias in the probe tests, whereas by the present example the effect was most pronounced at the early stages of learning. Since activation of NMDA receptors plays an important role in fast learning of several simultaneous aspects of complex tasks such as the Morris water maze (e.g., learning that there is a platform to provide escape from the water, that there is no escape through the wall, and determining the location of the platform with respect to extra-maze spatial cues), improved learning observed in the early phase of water maze acquisition in memantine-treated APP/PS1 mice compared to untreated transgenic mice indicates that the physiological functioning of NMDA receptors was restored by memantine under pathological conditions.

In the present example, memantine did not increase aggressive behavior in either APP/PS1 or NT mice.

Thus, subchronic oral administration of memantine mimicking its clinical use improved the impaired spatial learning of APP/PS transgenic mice but did not affect the increased aggression or reduced exploratory activity observed in these mice.

## Claims

1. Use of a 1-aminocyclohexane derivative in the manufacture of a medicament for creating, preventing, arresting, delaying the onset of and/or reducing the risk of developing an amyloidopathy selected from diffuse Lewy body disease, Creutzfeldt-Jakob disease, familial amyloidosis of Finnish type, familial amyloidotic polyneuropathy and hereditary cerebral hemorrhage with amyloidosis of the Dutch type in a mammal.

2. Use as claimed in claim 1 wherein the 1-aminocyclohexane derivative is a compound of general formula (I): 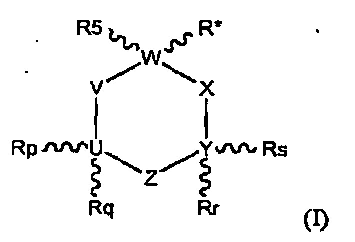 wherein:
- R^{*} is -(A)ₙ-(CR¹R²)ₘ-NR³R⁴,
n+m=0,1, or 2,
A is selected from linear or branched C₁₋₆ alkyl, linear or branched C₂₋₆ alkenyl and linear or branched C₂₋₆ alkynyl,
R¹ and R² are independently selected from hydrogen, linear or branched C₁₋₆ alkyl, linear or branched C₂₋₆ alkenyl, linear or branched C₂₋₆ alkynyl, aryl, substituted aryl and arylalkyl,
R³ and R⁴ are independently selected from hydrogen, linear or branched C₁₋₆ alkyl, linear or branched C₂₋₆ alkenyl and linear or branched C₂₋₆ alkynyl, or together form C₂₋₁₀ alkylene or C₂₋₁₀ alkenylene or together with the N form an optionally C₁₋₆ alkyl- or C₂₋₆ alkenyl-substituted 3-7-membered azacycloalkane or azacycloalkene; or independently R³ or R⁴ may join with R^{p}, R^{q}, R^{r} or R^{S} to form an alkylene chain -CH(R⁶)-(CH₂)ₜ- wherein t= 0 or 1 and the left side of the alkylene chain is attached to U or Y and the right side of the alkylene chain is attached to N, and R⁶ is selected from hydrogen, linear or branched C₁₋₆ alkyl, linear or branched C₂₋₆ alkenyl, linear or branched C₂₋₆ alkynyl, aryl, substituted aryl and arylalkyl; or independently R³ or R⁴ may join with R⁵ to form an alkylene chain represented by the formula -CH₂-CH₂-CH₂(CH₂)ᵣ, or an alkenylene chain represented by the formulae -CH=CH-CH₂-(CH₂)ₜ-, -CH=C=CH-(CH₂)ₜ- or -CH2-CH=CH-(CH₂)ₜ-, wherein t= 0 or 1, and the left side of the alkylene or alkenylene chain is attached to W and the right side of the chain is attached to N,
- R⁵ is independently selected from hydrogen, linear or branched C₁₋₆ alkyl, linear or branched C₂₋₆ alkenyl and linear or branched C₂₋₆ alkynyl, or R⁵ combines with the carbon to which it is attached and the next adjacent ring carbon to form a double bond,
- R^{p}, R^{q}, R^{r} and R^{s} are independently selected from hydrogen, linear or branched C₁₋₆ alkyl, linear or branched C₂₋₆ alkenyl, linear or branched C₂₋₆ alkynyl, C₃₋₆ cycloalkyl, aryl, substituted aryl and arylalkyl, or R^{p}, R^{q}, R^{r} and R^{s} independently may form a double bond with U or with Y or to which it is attached, or R^{p}, R^{q}, R^{r} and R^{s} may combine together to represent a C₂₋₅ alkylene or alkenylene bridge which may in turn combine with R⁵ to form an additional C₁₋₃ alkylene or alkenylene bridge,
- and the symbols U, V, W, X, Y and Z represent carbon atoms,
or an optical isomer, diastereomer, polymorph, enantiomer, hydrate, pharmaceutically acceptable salt therof or a mixture of compounds of formula (I).

3. Use as claimed in claim 1 wherein the 1-aminocyclohexane derivative is a compound of formula 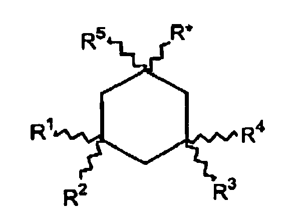 wherein R* is -(CH₂)*ₙ*-(CR⁶R⁷)*ₘ*-NR⁸R⁹,
n+m= 0, 1 or 2,
R¹ through R⁷ are independently selected from hydrogen and C₁₋₆ alkyl, at least R¹, R⁴ and R⁵ being C₁₋₆ alkyl, and R⁸ and R⁹ are independently selected from hydrogen and C₁₋₆ alkyl or together represent C₂₋₅ alkylene,
or an enantiomer, optical isomer, hydrate or pharmaceutically acceptable salt thereof.

4. Use as claimed in claim 1 wherein the 1-aminocyclohexane derivative is a compound of formula 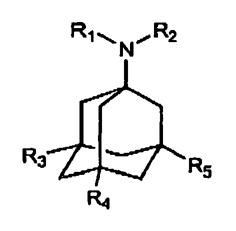 wherein R₁ and R₂ are the same or different and represent hydrogen or a straight or branched C₁₋₆ alkyl group or, in conjunction with N, a heterocyclic group with 5 or 6 ring C atoms,
R₃ and R₄ are the same or different and are selected from hydrogen, a straight or branched C₁₋₆ alkyl group, a C₅₋₆ cycloalkyl group and phenyl,
and R₅ is hydrogen or a straight or branched C₁₋₆ alkyl group,
or a pharmaceutically acceptable salt thereof.

5. Use as claimed in claim 1 wherein the 1-aminocyclohexane derivative is a compound of formula 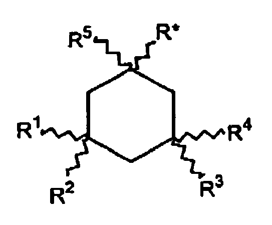 wherein R* is -(CH₂)*ₙ*-CR⁶R⁷)*ₘ*-NR⁸R⁹,
n+m=0, 1 or 2,
R¹ through R⁷ are independently selected from hydrogen, straight or branched C₁₋₆ alkyl, -CH₂- and C₅₋₆ cycloalkyl, at least R¹**,** R⁴ and R⁵ being C₁₋₆ alkyl or CH₂-,
and R⁸ and R⁹ are independently selected from hydrogen, straight or branched C₁₋₆ alkyl and C₅₋₆ cycloalkyl, or together represent C₂₋₅ alkylene or, in conjunction with N, represent a heterocyclic group with 5 or 6 ring C atoms,
provided that when R¹, R⁴ and R⁵ are each independently -CH₂-
R¹, R⁴ and R⁵ are each bonded to a single CR^{a} group to form a bridge, wherein R^{a} is selected from hydrogen, a straight or branched C₁₋₆ alkyl group, a C₅₋₆ cycloalkyl group and phenyl,
R² is selected from hydrogen, a straight or branched C₁₋₆ alkyl group, a C₅₋₆ cycloalkyl group and phenyl,
R³ is hydrogen or a straight or branched C₁₋₆ alkyl group, and
R^{*} is -(CH₂)*ₙ*-(CR⁶R⁷)*ₘ*-NR⁸R⁹, wherein n+m=0 and R⁸ and R⁹ are the same or different and represent hydrogen or a straight or branched C₁₋₆ alkyl group or, in conjunction with N, a heterocyclic group with 5 or 6 ring C atoms,
or an enantiomer, optical isomer, hydrate or pharmaceutically acceptable salt thereof

6. Use as claimed in claim 1 wherein the 1-aminocyclohexane derivative is selected from;
1-aminoadamantane,
1-amino-3-phenyladamantane,
1-aminomethyladamantane,
1-amino-3,5-dimethyladamantane (memantine),
1-amino-3-ethyladamantane,
1-amino-3-isopropyladamantane,
1-amino-3-n-butyladamantane,
1-amino-3,5-diethyladamantane,
1-amino-3,5-diisopropyladamantane,
1-amino-3,5-di-n-butyladamantane,
1-amino-3-methyl-5-ethyladamantane,
1-N-methylamino-3,5-dimethyladamantane,
1-N-ethylamino-3,5-dimethyladamantane,
1-N-isopropylamino-3,5-dimethyladamantane,
1-N,N-dimethylamino-3,5-dimethyladamantane,
1-N-methyl-N-isopropylamino-3-methyl-5-ethyladamantane,
1-amino-3-butyl-5-phenyladamantane,
1-amino-3-pentyladamantane,
1-amino-3,5-dipentyladamantane,
1-amino-3-pentyl-5-hexyladamantane,
1-amino-3-pentyl-5-cylohexyladamantane,
1-amino-3-pentyl-5-phenyladamantane,
1-amino-3-hexyladamantane,
1-amino-3,5-dihexyladamantane,
1-amino-3-hexyl-5-cyclohexyladamantane,
1-amino-3-hexyl-5-phenyladamantane,
1-amino-3-cyclohexyladamantane,
1-amino-3,5-dicyclohexyladamantane,
1-amino-3-cyclohexyl-5-phenyladamantane,
1-amino-3,5-diphenyladamantane,
1-amino-3,5,7-trimethyladamantane,
1-amino-3,5-dimethyl-7-ethyladamantane,
1-amino-3,5-diethyl-7-methyladamantane,
1-amino-3-methyl-5-propyladamantane,
1-amino-3-methyl-5-butyladamantane,
1-amino-3-methyl-5-pentyladamantane,
1-amino-3-methyl-5-hexyladamantane,
1-amino-3-methyl-5-cyclohexyladamantane,
1-amino-3-methyl-5-phenyladamantane,
1-amino-3-ethyl-5-propyladamantane,
1-amino-3-ethyl-5-butyladamantane,
1-amino-3-ethyl-5-pentyladamantane,
1-amino-3-ethyl-5-hexyladamantane,
1-amino-3-ethyl-5-cyclohexyladamantane,
1-amino-3-ethyl-5-phenyladamantane,
1-amino-3-propyl-5-butyladamantane,
1-amino-3-propyl-5-pentyladamantane,
1-amino-3-propyl-5-hexyladamantane,
1-amino-3-propyl-5-cyclohexyladamantane,
1-amino-3-propyl-5-phenyladamantane,
1-amino-3-butyl-5-pentyladamantane,
1-amino-3-butyl-5-hexyladamantane,
1-amino-3-butyl-5-cyclohexyladamantane,
and optical isomers, diastereomers, enantiomers, hydrates, N-methyl, N,N-dimethyl, N-ethyl, N-propyl, N-pyrrolidino and N-piperidino derivatives, pharmaceutically acceptable salts and mixtures thereof

7. Use as claimed in claim 1 wherein the 1-aminocyclohexane derivative is selected from memantine and prodrugs, salts, isomers, analogs and derivatives thereof.

8. Use as claimed in claim 7 wherein the 1-aminocyclohexane derivative is memantine.

9. Use as claimed in claim 1 wherein the 1-aminocyclohexane derivative is selected from:
1-amino-1,3,5-trimethylcyclohexane,
1-amino-1(trans),3(trans),5-trimethylcyclohexane,
1-amino-1(cis),3(cis),5-trimethylcyclohexane,
1-amino-1,3,3,5-tetramethylcyclohexane,
1-amino-1,3,3,5,5-pentamethylcyclohexane (neramexane), 1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcyclohexane,
1-amino-1-ethyl-3,3,5,5-tetramethylcyclohexane,
1-amino-1-propyl-3,3,5,5-tetramethylcyclohexane,
N-methyl-1-amino-1,3,3,5,5-pentamethylcyclohexane,
N-ethyl-1-amino-1,3,3,5,5-pentamethylcyclohexane,
N-(1,3,3,5,5-pentamethylcyclohexyl) pyrrolidine,
3,3,5,5-tetramethylcyclohexylmethylamine,
1-amino-1-propyl-3,3,5,5-tetramethylcyclohexane,
1 amino-1,3,3,5(trans)-tetramethylcyclohexane (axial amino group),
3-propyl-1,3,5,5-tetramethylcyclohexylamine semihydrate,
1-amino-1,3,5,5-tetramethyl-3-ethylcyclohexane,
1-amino-1,3,5-trimethylcyclohexane,
1-amino-1,3-dimethyl-3-propylcyclohexane,
1-amino-1,3(trans),5(trans)-trimethyl-3(cis)-propylcyclohexane,
1-amino-1,3-dimethyl-3-ethylcyclohexane,
1-amino-1,3,3-trimethylcyclohexane,
cis-3-ethyl-1(trans),3(trans),5-trimethylcyclohexamine,
1-amino-1,3 (trans)-dimethylcyclohexane,
1,3,3-trimethyl-5,5-dipropylcyclohexylamine,
1-amino-1-methyl-3(trans)-propylcyclohexane,
1-methyl-3(cis)-propylcyclohexylamine,
1-amino-1-methyl-3(trans)-ethylcyclohexane,
1-amino-1,3,3-trimethyl-5(cis)-ethylcyclohexane,
1-amino-1,3,3-trimethyl-5(trans)-ethylcyclohexane,
cis-3-propyl-1,5,5-trimethylcyclohexylamine,
trans-3-propyl-1,5,5-trimethylcyclohexylamine,
N-ethyl-1,3,3,5,5-pentamethylcyclohexylamine,
N-methyt-1-amino-1,3,3,5.5-pentamethylcyclohexane,
1-amino-1-methylcyclohexane,
N,N-dimethyl-1-amino-1,3,3,5,5-pentamethylcyclohexane,
2-(3,3,5,5-tetramethylcyclohexyl)ethylamine,
2-methyl-1-(3,3,5,5-tetramethylcyclohexyl)propyl-2-amine,
2-(1,3,3,5,5-pentamethylcyclohexyl)ethylamine semihydrate,
N-(1,3,3,5,5-pentamethylcyclohexyl)pyrrolidine,
1-amino-1,3(trans),5(trans)-trimethylcyclohexane,
1-amino-1,3(cis),5(cis)-trimethylcyclohexane,
1-amino-(1R,5S)trans-5-ethyl-1,3,3-trimethylcyclohexane,
1-amino-(1S,5S)cis-5-ethyl-1,3,3-trimethylcyclohexane,
1-amino-1,5,5-trimethyl-3(cis)-isopropylcyclohexane,
1-amino-1,5,5-trimethyl-3(trans)-isopropylcyclohexane,
1-amino-1-methyl-3(cis)-ethylcyclohexane,
1-amino-1-methyl-3(cis)-methylcyclohexane,
1-amino-5,5-diethyl-1,3,3-trimethylcyclohexane,
1-amino-1,3,3,5,5-pentamethylcyclohexane,
1-amino-1,5,5-trimethyl-3,3-diethylcyclohexanee,
1-amino-1-ethyl-3,3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylcyclohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,55)cis-5-ethyl,1,3,3-trimethylcyclohexyl]pyrrolidino or piperidine,
N-(1,3,3-trimethyl-trans-5-ethylcyclohexyl)pyrrolidine or piperidine,
N-[(1R,5S)trans-5-ethyl,3,3-trimethylcyclohexyl]pyrrolidine or piperidine,
N-(1-ethyl-3,3,5,5-tetramethylyclohexyl)pyrrolidine or piperidine,
N-(1-propyl-3,3,5,5-tetramethylcyclohexyl)pyrrolidine or piperidine,
N-(1,3,3,5,5-pentamethylcyclohexyl)pyrrolidine,
and optical isomers, diastereomers, enantiomers, hydrates, pharmaceutically acceptable salts and mixtures thereof.

10. Use as claimed in claim 1 wherein the 1-aminocyclohexane derivative is selected from neramexane and prodrugs, salts, isomers, analogs and derivatives thereof.

11. Use as claimed in claim 10 wherein the 1-aminocyclohexane derivative is neramexane.

12. Use as claimed in any preceding claim wherein the mammal is human.

13. Use as claimed in any preceding claim wherein the medicament is manufactured for administration of the 1-aminocyclohexane derivative in a therapeutically effective amount.

14. Use as claimed in claim 13 wherein the amount is in the range 1-100 mg/day.

15. Use as claimed in claim 14 wherein the amount is in the range 5-60 mg/day.

16. Use as claimed in claim 15 wherein the amount is in the range 10-40 mg/day.

17. Use as claimed in any preceding claim wherein the medicament further comprises a pharmaceutically acceptable carrier or excipient.

18. Use as claimed in any preceding claim wherein the medicament is manufactured for administration of the 1-aminocyclohexane derivative simultaneously or sequentially with another 1-aminocyclohexane derivative, an acetylcholinesterase inhibitor, a secretase modifier or a combination thereof.

19. Use as claimed in claim 18 wherein the acetylcholinesterase inhibitor is selected from galantamine, tacrine, donepezil and rivastigmine.

## Patentansprüche

1. Verwendung eines 1-Aminocyclohexan-Derivats für die Herstellung eines Medikaments für die Behandlung, Vorbeugung, Arretierung, Verzögerung des Ausbruchs einer Amyloidopathie und/oder für die Verringerung des Risikos, eine Amyloidopathie zu entwickeln, wobei die Amyloidopathie ausgewählt ist aus der diffusen Lewy-Body-Erkrankung, der Creutzfeldt-Jakob-Erkrankung, der familiären Amyloidose vom finnischen Typ, der familiären amyloiden Polyneuropathie und der erblichen cerebralen Hämorrhagie mit Amyloidose vom holländischen Typ in einem Säuger.

2. Verwendung gemäß Anspruch 1, wobei das 1-Aminocyclohexan-Derivat eine Verbindung der allgemeinen Formel (I) 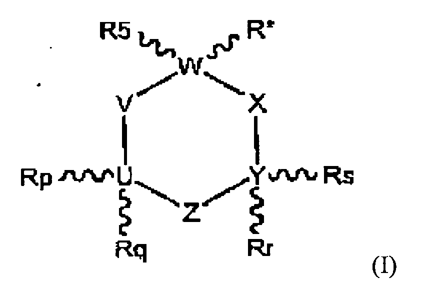 ist,
wobei
- R* -(A)ₙ-(CR¹R²)ₘ-NR³R⁴ ist,
n + m = 0, 1 oder 2 ist,
A aus linearem oder verzweigtem C₁₋₆-Alkyl, linearem oder verzweigtem C₂₋₆-Alkenyl und linearem oder verzweigtem C₂-₆-Alkynyl ausgewählt ist,
R¹ and R² unabhängig voneinander aus Wasserstoff, linearem oder verzweigtem C₁₋₆-Alkyl, linearem oder verzweigtem C₂-₆-Alkenyl, linearem oder verzweigtem C₂-₆-Alkynyl, Aryl, substituiertem Aryl und Arylalkyl ausgewählt sind,
R³ und R⁴ unabhängig voneinander aus Wasserstoff, linearem oder verzweigtem C₁-₆-Alkyl, linearem oder verzweigtem C₂-₆-Alkenyl, linearem oder verzweigtem C₂₋₆-Alkynyl ausgewählt sind oder zusammen C₂₋₁₀-Alkylen oder C₂₋₁₀-Alkenylen ausbilden oder zusammen mit dem N ein optional mit C₁₋₆-Alkyl oder C₂₋₆-Alkenyl substituiertes 3-7-gliedriges Azacycloalkan oder Azacycloalken ausbilden;
oder R³ oder R⁴ können sich unabhängig mit R^{p}, R^{q}, R^{r} oder R^{s} verbinden, so dass eine Alkylenkette -CH (R⁶)-(CH₂)ₜ- ausgebildet wird, wobei t = 0 oder 1 und die linke Seite der Alkylenkette an U oder Y angebracht ist und die rechte Seite der Alkylenkette an N angebracht ist und R⁶ aus Wasserstoff, linearem oder verzweigtem C₁₋₆-Alkyl, linearem oder verzweigtem C₂-₆-Alkenyl, linearem oder verzweigtem C₂-₆-Alkynyl, Aryl, substituiertem Aryl und Arylalkyl ausgewählt sind;
oder R³ oder R⁴ können sich unabhängig voneinander mit R⁵ verbinden, so dass eine Alkylenkette, die durch die Formel -CH₂-CH₂-CH₂-(CH₂)ₜ-dargestellt wird, oder eine Alkenylenkette, die durch die Formeln -CH=CH-CH₂-(CH₂)ᵣ, -CH=C=CH-(CH2)ₜ- oder -CH2-CH=CH-(CH2)-dargestellt wird, ausgebildet wird, wobei t = 0 oder 1 und die linke Seite der Alkylen- oder Alkenylenkette an W angebracht ist und die rechte Seite der Kette an N angebracht ist;
- R⁵ unabhängig aus Wasserstoff, linearem oder verzweigtem C₁₋₆-Alkyl, linearem oder verzweigtem C₂₋₆-Alkenyl, linearem oder verzweigtem C₂₋₆-Alkynyl ausgewählt ist, oder R⁵ sich mit dem Kohlenstoff, an das er angebracht ist, und dem nächsten benachbarten Ringkohlenstoff verbindet, so dass eine Doppelbindung ausgebildet wird,
- R^{P}, R^{q}, R^{r} und R^{s} unabhängig voneinander aus Wasserstoff, linearem oder verzweigtem C₁₋₆-Alkyl, linearem oder verzweigtem C₂₋₆-Alkenyl, linearem oder verzweigtem C₂₋₆-Alkynyl, C₃₋₆-Cycloalkyl, Aryl, substituiertem Aryl und Arylalkyl ausgewählt sind, oder R^{p}, R^{q}, R^{r} und R^{s} unabhängig voneinander eine Doppelbindung mit U oder mit Y oder mit dem, an welchen sie angebracht sind, ausbilden können, oder R^{P}, R^{q}, R^{r} und R^{s} sich miteinander verbinden können, so dass sie eine C₂₋₅-Alkylen- oder Alkenylen-Brücke darstellen, die sich im Gegenzug mit R⁵ verbinden kann, so dass eine zusätzliche C₁₋₃-Alkylen- oder Alkenylen-Brücke ausgebildet wird,
- und die Symbole U, V, W, X, Y und Z Kohlenstoffatome darstellen,
oder ein optisches Isomer, Diastereomer, Polymorph, Enantiomer, Hydrat, pharmazeutisch verträgliches Salz davon oder ein Gemisch von Verbindungen der Formel (I) ist.

3. Verwendung gemäß Anspruch 1, wobei das 1-Aminocyclohexan-Derivat eine Verbindung der Formel 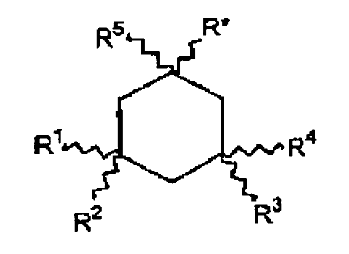 ist,
wobei
R^{*} -(CH₂)ₙ-(CR⁶R⁷)ₘ-NR⁸R⁹ ist,
n + m = 0, 1 oder 2 ist,
R¹ bis R⁷ allesamt unabhängig voneinander aus Wasserstoff und C₁₋₆-Alkyl ausgewählt sind, wobei wenigstens R¹, R⁴ und R⁵ C₁₋₆-Alkyl sind und R⁸ und R⁹ unabhängig voneinander aus Wasserstoff und C₁₋₆-Alkyl ausgewählt sind oder zusammen C₂₋₅-Alkylen darstellen,
oder ein Enantiomer, optisches Isomer, Hydrat oder pharmazeutisch verträgliches Salz davon ist.

4. Verwendung gemäß Anspruch 1, wobei das 1-Aminocyclohexan-Derivat eine Verbindung der Formel 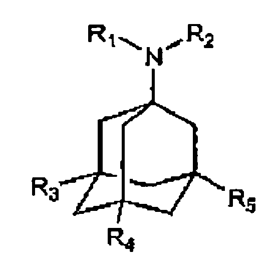 ist,
wobei
R₁ und R₂ gleich oder unterschiedlich sind und Wasserstoff oder eine gerade oder verzweigte C₁₋₆-Alkyl-Gruppe oder, in Verbindung mit N, eine heterocyclische Gruppe mit 5 oder 6 Ring-C-Atomen darstellen,
R₃ und R₄ gleich oder unterschiedlich sind und aus Wasserstoff, einer geraden oder verzweigten C₁₋₆-Alkyl-Gruppe, einer C₅₋₆-Cycloalkyl-Gruppe und Phenyl ausgewählt sind
und R₅ Wasserstoff oder eine gerade oder verzweigte C₁₋₆-Alkyl-Gruppe ist,
oder ein pharmazeutisch verträgliches Salz davon ist.

5. Verwendung gemäß Anspruch 1, wobei das 1-Aminocyclohexan-Derivat eine Verbindung der Formel 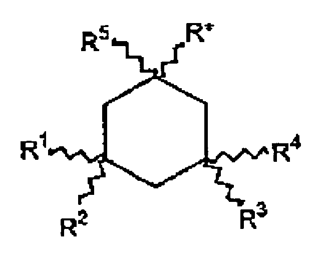 ist,
wobei
R^{*} -(CH₂)ₙ-(CR⁶R⁷)ₘ-NR⁸R⁹ ist,
n + m = 0, 1 oder 2 ist,
R¹ bis R⁷ allesamt unabhängig voneinander aus Wasserstoff, geradem oder verzweigtem C₁₋₆-Alkyl, -CH₂- und C₅₋₆-Cycloalkyl ausgewählt sind, wobei wenigstens R¹, R⁴ und R⁵ C₁₋₆-Alkyl oder -CH₂- sind,
und R⁸ und R⁹ unabhängig voneinander aus Wasserstoff, geradem oder verzweigtem C₁₋₆-Alkyl und C₅₋₆-Cycloalkyl ausgewählt sind, oder zusammen C₂₋₅-Alkylen darstellen oder, in Verbindung mit N, eine heterocyclische Gruppe mit 5 oder 6 Ring-C-Atomen darstellen,
vorausgesetzt, dass, wenn R¹, R⁴ und R⁵ jeweils unabhängig -CH₂- sind,
R¹, R⁴ und R⁵ jeweils an eine einzelne CR^{a}-Gruppe gebunden sind, so dass eine Brücke ausgebildet wird, wobei R^{a} aus Wasserstoff, einer geraden oder verzweigten C₁₋₆-Alkyl-Gruppe, einer C₅₋₆-Cycloalkyl-Gruppe und Phenyl ausgewählt ist,
R² aus Wasserstoff, einer geraden oder verzweigten C₁₋₆-Alkyl-Gruppe, einer C₅₋₆-Cycloalkyl-Gruppe und Phenyl ausgewählt ist,
R³ Wasserstoff oder eine gerade oder verzweigte C₁₋₆-Alkyl-Gruppe ist, und
R* -(CH₂)ₙ-(CR⁶R⁷)ₘ-NR⁸R⁹ ist, wobei n + m = 0 ist und R⁸ und R⁹ gleich oder unterschiedlich sind und Wasserstoff oder eine gerade oder verzweigte C₁₋₆-AlkylGruppe oder, in Verbindung mit N, eine heterocyclische Gruppe mit 5 oder 6 Ring-C-Atomen darstellen,
oder ein Enantiomer, optisches Isomer, Hydrat oder pharmazeutisch verträgliches Salz davon ist.

6. Verwendung gemäß Anspruch 1, wobei das 1-Aminocyclohexan-Derivat ausgewählt ist aus:
1-Aminoadamantan,
1-Amino-3-Phenyladamantan,
1 -Aminomethyladamantan,
1-Amino-3,5-Dimethyladamantan (Memantin),
1-Amino-3-Ethyladamantan,
1-Amino-3-Isopropyladamantan,
1-Amino-3-*n*-Butyladamantan,
1-Amino-3,5-Diethyladamantan,
1-Amino-3,5-Diisopropyladamantan,
1-Amino-3,5-Di-*n*-Butyladamantan,
1-Amino-3-Methyl-5-Ethyladamantan,
1-N-Methylamino-3,5-Dimethyladamantan,
1-N-Ethylamino-3,5-Dimethyladamantan,
1-N-Isopropylamino-3,5-Dimethyladamantan,
1 -N,N-Dimethylamino-3,5-Dimethyladamantan,
1-N-Methyl-N-Isopropylamino-3-Methyl-5-Ethyladamantan,
1-Amino-3-Butyl-5-Phenyladamantan,
1-Amino-3-Pentyladamantan,
1-Amino-3,5-Dipentyladamantan,
1-Amino-3-Pentyl-5-Hexyladamantan,
1-Amino-3-Pentyl-5-Cyclohexyladamantan,
1-Amino-3-Pentyl-5-Phenyladamantan,
1-Amino-3-Hexyladamantan,
1-Amino-3,5-Dihexyladamantan,
1-Amino-3-Hexyl-5-Cyclohexyladamantan,
1-Amino-3-Hexyl-5-Phenyladamantan,
1-Amino-3-Cyclohexyladamantan,
1-Amino-3,5-Dicyclohexyladamantan,
1-Amino-3-Cyclohexyl-5-Phenyladamantan,
1-Amino-3,5-Diphenyladamantan,
1-Amino-3,5,7-Trimethyladamantan,
1-Amino-3,5-Dimethyl-7-Ethyladamantan,
1-Amino-3,5-Diethyl-7-Methyladamantan,
1-Amino-3-Methyl-5-Propyladamantan,
1-Amino-3-Methyl-5-Butyladamantan,
1-Amino-3-Methyl-5-Pentyladamantan,
1-Amino-3-Methyl-5-Hexyladamantan,
1-Amino-3-Methyl-5-Cyclohexyladamantan,
1-Amino-3-Methyl-5-Phenyladamantan,
1-Amino-3-Ethyl-5-Propyladamantan,
1-Amino-3-Ethyl-5-Butyladamantan,
1-Amino-3-Ethyl-5-Pentyladamantan,
1-Amino-3-Ethyl-5-Hexyladamantan,
1-Amino-3-Ethyl-5-Cyclohexyladamantan,
1-Amino-3-Ethyl-5-Phenyladamantan,
1-Amino-3-Propyl-5-Butyladamantan,
1-Amino-3-Propyl-5-Pentyladamantan,
1-Amino-3-Propyl-5-Hexyladamantan,
1 -Amino-3-Propyl-5-Cyclohexyladamantan,
1-Amino-3-Propyl-5-Phenyladamantan,
1-Amino-3-Butyl-5-Pentyladamantan,
1-Amino-3-Butyl-5-Hexyladamantan,
1-Amino-3-Butyl-5-Cyclohexyladamantan,
und optischen Isomeren, Diastereomeren, Enantiomeren, Hydraten, N-Methyl-, N,N-Dimethyl-, N-Ethyl-, N-Propyl-, N-Pyrrolidino- und N-Piperidinoderivaten, pharmazeutisch verträglichen Salzen und Gemischen davon.

7. Verwendung gemäß Anspruch 1, wobei das 1-Aminocyclohexan-Derivat aus Memantin und Pro-Pharmaka (*prodrugs*), Salzen, Isomeren, Analoga und Derivaten davon ausgewählt ist.

8. Verwendung gemäß Anspruch 7, wobei das 1-Aminocyclohexan-Derivat Memantin ist.

9. Verwendung gemäß Anspruch 1, wobei das 1-Aminocyclohexan-Derivat ausgewählt ist aus:
1-Amino-1,3,5-Trimethylcyclohexan,
1-Amino-1 (*trans*),3 (*trans*),5-Trimethylcyclohexan,
1-Amino-1 (*cis*),3 (*cis*),5-Trimethylcyclohexan,
1-Amino-1,3,3,5-Tetramethylcyclohexan,
1-Amino-1,3,3,5,5-Pentamethylcyclohexan (Neramexan),
1-Amino-1,3,5,5-Tetramethyl-3-Ethylcyclohexan,
1-Amino-1,5,5-Trimethyl-3,3-Diethylcyclohexan,
1-Amino-1,5,5-Trimethyl-*cis*-3-Ethylcyclohexan,
1-Amino-(1 S, 5 S) c*is*-3-Ethyl-1,5,5-Trimethylcyclohexan,
1-Amino-1,5,5-Trimethyl-*trans*-3-Ethylcyclohexan,
1-Amino- (1 R, 5 S) *trans*-3-Ethyl-1,5,5-Trimethylcyclohexan,
1-Amino-1-Ethyl-3,3,5,5-Tetramethylcyclohexan,
1-Amino-1-Propyl-3,3,5,5-Tetramethylcyclohexan,
N-Methyl-1-Amino-1,3,3,5,5-Pentamethylcyclohexan,
N-Ethyl-1-Amino-1,3,3,5,5-Pentamethylcyclohexan,
N-(1,3,3,5,5-Pentamethylcyclohexyl)-Pyrrolidin,
3,3,5,5-Tetramethylcyclohexylmethylamin,
1-Amino-1-Propyl-3,3,5,5-Tetramethylcyclohexan,
1-Amino-1,3,3,5 (*trans*)-Tetramethylcyclohexan (axiale Aminogruppe),
3-Propyl-1,3,5,5-Tetramethylcyclohexylamin-Semihydrat,
1-Amino-1,3,5,5-Tetramethyl-3-Ethylcyclohexan,
1-Amino-1,3,5-Trimethylcyclohexan,
1-Amino-1,3-Dimethyl-3-Propylcyclohexan,
1-Amino-1,3(*trans*),5 (*trans*)-Trimethyl-3(*cis*)-Propylcyclohexan,
1-Amino-1,3-Dimethyl-3-Ethylcyclohexan,
1-Amino-1,3,3-Trimethylcyclohexan,
cis-3-Ethyl-1(*trans*)*-*3 (*trans*)-5-Trimethylcyclohexamin,
1-Amino-1,3 (*trans*)-Dimethylcyclohexan,
1,3,3-Trimethyl-5,5-Dipropylcyclohexylamin,
1-Amino-1-Methyl-3 (*trans*)-Propylcyclohexan,
1-Methyl-3 (*cis*)-Propylcyclohexylamin,
1-Amino-1-Methyl-3 (*trans*)-Ethylcyclohexan,
1-Amino-1,3,3-Trimethyl-5 (*cis*)-Ethylcyclohexan,
1-Amino-1,3,3-Trimethyl-5 (*trans*)-Ethylcyclohexan,
cis-3-Propyl-1,5,5-Trimethylcyclohexylamin,
trans-3-Propyl-1,5,5-Trimethylcyclohexylamin,
N-Ethyl-1,3,3,5,5-Pentamethylcyclohexylamin,
N-Methyl-1-Amino-1,3,3,5,5-Pentamethylcyclohexan,
1-Amino-1-Methylcyclohexan,
N,N-Dimethyl-1-Amino-1,3,3,5,5-Pentamethylcyclohexan,
2-(3,3,5,5-Tetramethylcyclohexyl)-Ethylamin,
2-Methyl-1-(3,3,5,5-Tetramethylcyclohexyl)-Propyl-2-Amin,
2-(1,3,3,5,5-Pentamethylcyclohexyl)-Ethylamin-Semihydrat,
N-(1,3,3,5,5-Pentamethylcyclohexyl)-Pyrrolidin,
1-Amino-1,3 (*trans*)*,* 5 (*trans*)-Trimethylcyclohexan,
1-Amino-1,3 (*cis*)*,* 5 (*cis*)-Trimethylcyclohexan,
1-Amino-(1 R, 5 S) *trans*-5-Ethyl-1,3,3-Trimethylcyclohexan,
1-Amino-(1 S, 5 S) *cis*-5-Ethyl-1,3,3-Trimethylcyclohexan,
1-Amino-1,5,5-Trimethyl-3 (*cis*)-Isopropylcyclohexan,
1-Amino-1,5,5-Trimethyl-3 (*trans*)-Isopropylcyclohexan,
1-Amino-1-Methyl-3 (*cis*)-Ethylcyclohexan,
1-Amino-1-Methyl-3 (*cis*)-Methylcyclohexan,
1-Amino-5,5-Diethyl-1,3,3-Trimethylcyclohexan,
1-Amino-1,3,3,5,5-Pentamethylcyclohexan,
1-Amino-1,5,5-Trimethyl-3,3-Diethylcyclohexan,
1-Amino-1-Ethyl-3,3,5,5-Tetramethylcyclohexan,
N-Ethyl-1-Amino-1,3,3,5,5-Pentamethylcyclohexan,
N-(1,3,5-Trimethylcyclohexyl)-Pyrrolidin oder -Piperidin,
N-[1,3 *(trans),* 5 (*trans*)-Trimethylcyclohexyl]-Pyrrolidin oder -Piperidin,
N-[1,3 *(cis),* 5 (*cis*)-Trimethylcyclohexyl]-Pyrrolidin oder -Piperidin,
N-(1,3,3,5-Tetramethylcyclohexyl)-Pyrrolidin oder -Piperidin,
N-(1,3,3,5,5-Pentamethylcyclohexyl)-Pyrrolidin oder -Piperidin,
N-(1,3,5,5-Tetramethyl-3-Ethylcyclohexyl)-Pyrrolidin oder -Piperidin,
N-(1,5,5-Trimethyl-3,3-Diethylcyclohexyl)-Pyrrolidin oder -Piperidin,
N-(1,3,3-Trimethyl-*cis*-5-Ethylcyclohexyl)-Pyrrolidin oder -Piperidin,
N-[(1 S, 5 S) *cis*-5-Ethyl-1,3,3-Trimethylcyclohexyl]-Pyrrolidin oder -Piperidin,
N-(1,3,3-Trimethyl-*trans*-5-Ethylcyclohexyl)-Pyrrolidin oder Piperidin,
N-[(1R,5 S) *trans*-5-Ethyl-3,3-Trimethylcyclohexyl]-Pyrrolidin oder -Piperidin,
N-(1-Ethyl-3,3,5,5-Tetramethylyclohexyl)-Pyrrolidin oder -Piperidin,
N-(1-Propyl-3,3,5,5-Tetramethylcyclohexyl)-Pyrrolidin oder -Piperidin,
N-(1,3,3,5,5-Pentamethylcyclohexyl)-Pyrrolidin,
und optischen Isomeren, Diastereomeren, Enantiomeren, Hydraten, pharmazeutisch verträglichen Salzen und Gemischen davon.

10. Verwendung gemäß Anspruch 1, wobei das 1-Aminocyclohexan-Derivat aus Neramexan und Pro-Pharmaka (*prodrugs*)*,* Salzen, Isomeren, Analoga und Derivaten davon ausgewählt ist.

11. Verwendung gemäß Anspruch 10, wobei das 1-Aminocyclohexan-Derivat Neramexan ist.

12. Verwendung gemäß einem der vorangehenden Ansprüche, wobei der Säuger ein Mensch ist.

13. Verwendung gemäß einem der vorangehenden Ansprüche, wobei das Medikament hergestellt ist für die Verabreichung des 1-Aminocyclohexan-Derivats in einer therapeutisch wirksamen Menge.

14. Verwendung gemäß Anspruch 13, wobei die Menge im Bereich von 1 - 100 mg/Tag liegt.

15. Verwendung gemäß Anspruch 14, wobei die Menge im Bereich von 5 - 60 mg/Tag liegt.

16. Verwendung gemäß Anspruch 15, wobei die Menge im Bereich von 10 - 40 mg/Tag liegt.

17. Verwendung gemäß einem der vorangehenden Ansprüche, wobei das Medikament weiter einen pharmazeutisch verträglichen Träger oder Exzipienten umfasst.

18. Verwendung gemäß einem der vorangehenden Ansprüche, wobei das Medikament hergestellt ist für die gleichzeitige oder sequenzielle Verabreichung des 1-Aminocyclohexan-Derivats mit einem anderen 1-Aminocyclohexan-Derivat, einem Acetylcholinesterase-Inhibitor, einem Sekretase-Modifizierer oder einer Kombination davon.

19. Verwendung gemäß Anspruch 18, wobei der Acetylcholinesterase-Inhibitor aus Galantamin, Tacrin, Donepezil und Rivastigmin ausgewählt ist.

## Revendications

1. Utilisation d'un dérivé de 1-aminocyclohexane pour la fabrication d'un médicament pour traiter, empêcher arrêter, retarder la survenue de et/ou réduire le risque de développement d'une amyloïdopathie choisie par la maladie diffuse à corps de Lewy, la maladie de Creutzfeldt-Jakob, l'amyloïdose familiale de type finlandais, la polyneuropathie amyloïdotique familiale et l'hémorragie cérébrale héréditaire avec amyloïdose de type hollandais chez un mammifère.

2. Utilisation selon la revendication 1, dans laquelle le dérivé de 1-aminocyclohexane est un composé de formule générale (I) : 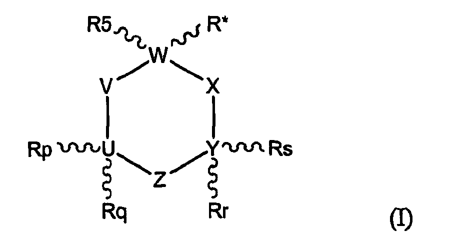 dans laquelle :
R* est -(A)ₙ(CR¹R²)ₘ-NR³R⁴,
n+m=0, 1,ou2,
A est choisi parmi les groupes alkyle en C₁₋₆ linéaire ou ramifié, alcényle en C₂₋₆ linéaire ou ramifié, et alcynyle en C₂₋₆ linéaire ou ramifié,
R¹ et R² sont indépendamment choisis parmi un atome d'hydrogène, les groupes alkyle en C₁₋₆ linéaire ou ramifié, alcényle en C₂₋₆ linéaire ou ramifié, alcynyle en C₂₋₆ linéaire ou ramifié, aryle, aryle substitué et arylalkyle,
R³ et R⁴ sont indépendamment choisis parmi un atome d'hydrogène, les groupes alkyle en C₁₋₆ linéaire ou ramifié, alcényle en C₂₋₆ linéaire ou ramifié, et alcynyle en C₂₋₆ linéaire ou ramifié, ou ensemble forment un groupe alkylène en C₂₋₁₀ ou alcénylène en C₂₋₁₀ ou ensemble avec N forment un groupe azacycloalcane ou azacycloalcène de 3 à 7 éléments éventuellement substitué par un groupe alkyle en C₁₋₆ ou alcényle en C₂₋₆ ; ou indépendamment R³ ou R⁴ peut être lié à R^{P}, R^{q}, R^{r} ou R^{s} pour former une chaîne alkylène -CH(R⁶)-(CH₂)ₜ- où t = 0 ou 1 et le côté gauche de la chaîne alkylène est attaché à U ou Y et le côté droit de la chaîne alkylène est attaché à N, et R⁶ est choisi parmi un atome d'hydrogène, les groupes alkyle en C₁₋₆ linéaire ou ramifié, alcényle en C₂₋₆ linéaire ou ramifié, alcynyle en C₂₋₆ linéaire ou ramifié, aryle, aryle substitué et arylalkyle ; ou indépendamment R³ ou R⁴ peut être lié à R⁵ pour former une chaîne alkylène représentée par la formule -CH₂-CH₂-CH₂-(CH₂)ₜ- ou une chaîne alcénylène représentée par la formule -CH=CH-CH₂-(CH₂)ₜ-, -CH=C=CH-(CH₂)ₜ- ou -CH₂-CH=CH-(CH₂)ₜ—, où t = 0 ou 1, et le côté gauche de la chaîne alkylène ou alcénylène est attaché à W et le côté droit de la chaîne est attaché à N,
R⁵ est indépendamment choisi parmi un atome d'hydrogène, les groupes alkyle en C₁₋₆ linéaire ou ramifié, alcényle en C₂₋₆ linéaire ou ramifié, et alcynyle en C₂₋₆ linéaire ou ramifié, ou R⁵ est combiné avec l'atome de carbone auquel il est attaché et l'atome de carbone du cycle adjacent suivant pour former une double liaison,
R^{p}, R^{q}, R^{r} et R^{s} sont indépendamment choisis parmi un atome d'hydrogène, les groupes alkyle en C₁₋₆ linéaire ou ramifié, alcényle en C₂₋₆ linéaire ou ramifié, alcynyle en C₂₋₆ linéaire ou ramifié, cycloalkyle en C₃-C₆, aryle, aryle substitué et arylalkyle, ou R^{p}, R^{q}, R^{r} et R^{s} peuvent indépendamment former une double liaison avec U ou avec Y ou avec celui auquel il est attaché, ou R^{p}, R^{q}, R^{r} et R^{s} peuvent être combinés ensemble pour représenter un pont alkylène ou alcénylène en C₂-C₅ qui peut à son tour être combiné avec R⁵ pour former un pont alkylène ou alcénylène en C₁₋₃ supplémentaire,
et les symboles U, V, W, X, Y et Z représentent des atomes de carbone,
ou un isomère optique, un diastéréoisomère, un polymorphe, un énantiomère, un hydrate, un sel pharmaceutiquement acceptable de celui-ci ou un mélange de composés de formule (I).

3. Utilisation selon la revendication 1, dans laquelle le dérivé de 1-aminocyclohexane est un composé de formule : 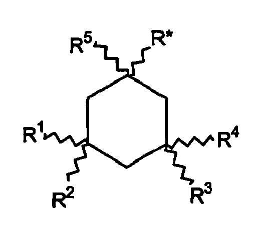 dans laquelle :
R* est -(CH₂)ₙ-(CR⁶R⁷)ₘ-NR⁸R⁹,
n+m=0,1, ou 2,
R¹ à R⁷ sont indépendamment choisis parmi un atome d'hydrogène et un groupe alkyle en C₁₋₆, au moins R¹, R⁴ et R⁵ étant un groupe alkyle en C₁₋₆, et R⁸ et R⁹ sont indépendamment choisis parmi un atome d'hydrogène et un groupe alkyle en C₁₋₆, ou ensemble représentent un groupe alkylène en C₂₋₅,
ou un énantiomère, un isomère optique, un hydrate ou un sel pharmaceutiquement acceptable de celui-ci.

4. Utilisation selon la revendication 1, dans laquelle le dérivé de 1-aminocyclohexane est un composé de formule : 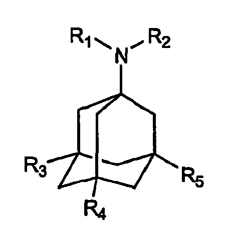 dans laquelle R₁ et R₂ sont identiques ou différents et représentent un atome d'hydrogène ou un groupe alkyle en C₁₋₆ linéaire ou ramifié, ou conjointement avec N, un groupe hétérocyclique ayant 5 ou 6 atomes de carbone sur le cycle,
R₃ et R₄ sont identiques ou différents et sont choisis parmi un atome d'hydrogène, un groupe alkyle en C₁₋₆ linéaire ou ramifié, un groupe cycloalkyle en C₅₋₆ et un groupe phényle,
et R₅ est un atome d'hydrogène ou un groupe alkyle en C₁₋₆ linéaire ou ramifié,
ou un sel pharmaceutiquement acceptable de celui-ci.

5. Utilisation selon la revendication 1, dans laquelle le dérivé de 1-aminocyclohexane est un composé de formule : 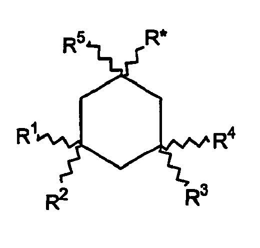 dans laquelle :
R* est -(CH₂)ₙ-(CR⁶R⁷)ₘ-NR⁸R⁹,
n+m=0, 1, ou 2,
R¹ à R⁷ sont indépendamment choisis parmi un atome d'hydrogène, un groupe alkyle en C₁₋₆ linéaire ou ramifié, -CH₂- et un groupe cycloalkyle en C₅₋₆, au moins R¹, R⁴ et R⁵ étant un groupe alkyle en C₁₋₆ ou -CH₂-,
et R⁸ et R⁹ sont indépendamment choisis parmi un atome d'hydrogène, un groupe alkyle en C₁₋₆ linéaire ou ramifié et un groupe cycloalkyle en C₅₋₆, ou ensemble représentent un groupe alkylène en C₂₋₅, ou conjointement avec N, représente un groupe hétérocyclique ayant 5 ou 6 atomes de carbone sur le cycle,
à condition que lorsque R¹, R⁴ et R⁵ sont chacun indépendamment -CH₂-
R¹, R⁴ et R⁵ soient chacun attachés à un groupe CR^{a} unique pour former un pont, dans lequel R^{a} est choisi parmi un atome d'hydrogène, un groupe alkyle en C₁₋₆ linéaire ou ramifié, un groupe cycloalkyle en C₅₋₆, et un groupe phényle,
R² est choisi parmi un atome d'hydrogène, un groupe alkyle en C₁₋₆ linéaire ou ramifié, un groupe cycloalkyle en C₅₋₆, et un groupe phényle,
R³ est un atome d'hydrogène ou un groupe alkyle en C₁₋₆ linéaire ou ramifié, et
R* est -(CH₂)ₙ-(CR⁶R⁷)ₘ-NR⁸R⁹, où n+m = 0 et R⁸ et R⁹ sont identiques ou différents et représentent un atome d'hydrogène ou un groupe alkyle en C₁₋₆ linéaire ou ramifié, ou conjointement avec N, un groupe hétérocyclique ayant 5 ou 6 atomes de carbone sur le cycle,
ou un énantiomère, un isomère optique, un hydrate ou un sel pharmaceutiquement acceptable de celui-ci.

6. Utilisation selon la revendication 1, dans laquelle le dérivé de 1-aminocyclohexane est choisi parmi :
le 1-aminoadamantane,
le 1-amino-3-phényladamantane,
le 1-aminométhyladamantane,
le 1-amino-3, 5-diméthyladamantane (mémantine),
le 1-amino-3-éthyladamantane,
le 1-amino-3-isopropyladamantane,
le 1-amino-3-n-butyladamantane,
le 1-amino-3,5-diéthyladamantane,
le 1-amino-3,5-diisopropyladamantane,
le 1-amino-3,5-di-n-butyladamantane,
le 1-amino-3-méthyl-5-éthyladamantane,
le 1-N-méthylamino-3,5-diméthyladamantane,
le 1-N-éthylamino-3,5-diméthyladamantane,
le 1-N-isopropylamino-3,5-diméthyladamantane,
le 1-N,N-diméthylamino-3,5-diméthyladamantane,
le 1-N-méthyl-N-isopropylamino-3-méthyl-5-éthyladamantane,
le 1-amino-3-butyl-5-phényladamantane,
le 1-amino-3-pentyladamantane,
le 1-amino-3,5-dipentyladamantane,
le 1-amino-3-pentyl-5-hexyladamantane,
le 1-amino-3-pentyl-5-cyclohexyladamantane,
le 1-amino-3-pentyl-5-phényladamantane,
le 1-amino-3-hexyladamantane,
le 1-amino-3,5-dihexyladamantane,
le 1-amino-3-hexyl-5-cyclohexyladamantane,
le 1-amino-3-hexyl-5-phényladamantane,
le 1-amino-3-cyclohexyladamantane,
le 1-amino-3,5-dicyclohexyladamantane,
le 1-amino-3-cyclohexyl-5-phényladamantane,
le 1-amino-3,5-diphényladamantane,
le 1-amino-3,5,7-triméthyladamantane,
le 1-amino-3,5-diméthyl-7-éthyladamantane,
le 1-amino-3,5-diéthyl-7-méthyladamantane,
le 1-amino-3-méthyl-5-propyladamantane,
le 1-amino-3-méthyl-5-butyladamantane,
le 1-amino-3-méthyl-5-pentyladamantane,
le 1-amino-3-méthyl-5-hexyladamantane,
le 1-amino-3-méthyl-5-cyclohexyladamantane,
le 1-amino-3-méthyl-5-phényladamantane,
le 1-amino-3-éthyl-5-propyladamantane,
le 1-amino-3-éthyl-5-butyladamantane,
le 1-amino-3-éthyl-5-pentyladamantane,
le 1-amino-3-éthyl-5-hexyladamantane,
le 1-amino-3-éthyl-5-cyclohexyladamantane,
le 1-amino-3-éthyl-5-phényladamantane,
le 1-amino-3-propyl-5-butyladamantane,
le 1-amino-3-propyl-5-pentyladamantane,
le 1-amino-3-propyl-5-hexyladamantane,
le 1-amino-3-propyl-5-cyclohexyladamantane,
le 1-amino-3-propyl-5-phényladamantane,
le 1-amino-3-butyl-5-pentyladamantane,
le 1-amino-3-butyl-5-hexyladamantane,
le 1-amino-3-butyl-5-cyclohexyladamantane,
et les isomères optiques, diastéréoisomères, énantiomères, hydrates, dérivés de N-méthyle, N,N-diméthyle, N-éthyle, N-propyle, N-pyrrolidino et N-pipéridino, sels pharmaceutiquement acceptables et mélanges de ceux-ci.

7. Utilisation selon la revendication 1, dans laquelle le dérivé de 1-aminocyclohexane est choisi parmi la mémantine et les pro-médicaments, sels, isomères, analogues et dérivés de celle-ci.

8. Utilisation selon la revendication 7, dans laquelle le dérivé de 1-aminocyclohexane est la mémantine.

9. Utilisation selon la revendication 1, dans laquelle le dérivé de 1-aminocyclohexane est choisi parmi :
le 1-amino-1,3,5-triméthylcyclohexane,
le 1-amino-1(trans),3(trans),5-triméthylcyclohexane,
le 1-amino-1(cis),3(cis),5-triméthylcyclohexane,
le 1-amino-1,3,3,5-tétraméthylcyclohexane,
le 1-amino-1,3,3,5,5-pentaméthylcyclohexane (néramexane),
le 1-amino-1,3,5,5-tétraméthyl-3-éthylcyclohexane,
le 1-amino-1,5,5-triméthyl-3,3-diéthylcyclohexane,
le 1-amino-1,5,5-triméthyl-cis-3-éthylcyclohexane,
le 1-amino-(1S,5S)cis-3-éthyl-1,5,5-triméthylcyclohexane,
le 1-amino-1,5,5-triméthyl-trans-3-éthylcyclohexane,
le 1-amino-(1R,5S)trans-3-éthyl-1,5,5-triméthylcyclohexane,
le 1-amino-1-éthyl-3,3,5,5-tétraméthylcyclohexane,
le 1-amino-1-propyl-3,3,5,5-tétraméthylcyclohexane,
le N-méthyl-1-amino-1,3,3,5,5-pentaméthylcyclohexane,
le N-éthyl-1-amino-1,3,3,5,5-pentaméthylcyclohexane,
la N-(1,3,3,5,5-pentaméthylcyclohexyl)pyrrolidine,
la 3,3,5,5-tétraméthylcyclohexylméthylamine,
le 1-amino-1-propyl-3,3,5,5-tétraméthylcyclohexane,
le 1-amino-1,3,3,5(trans)-tétraméthylcyclohexane (groupe amino axial),
le 3-propyl-1,3,5,5-tétraméthylcyclohexylamine semihydrate,
le 1-amino-1,3,5,5-tétraméthyl-3-éthylcyclohexane,
le 1-amino-1,3,5-triméthylcyclohexane,
le 1-amino-1,3-diméthyl-3-propylcyclohexane,
le 1-amino-1,3(trans),5(trans)-triméthyl-3(cis)-propylcyclohexane,
le 1-amino-1,3-diméthyl-3-éthylcyclohexane,
le 1-amino-1,3,3-triméthylcyclohexane,
le cis-3-éthyl-1(trans),3(trans)-5-triméthylcyclohexamine,
le 1-amino-1,3(trans)-diméthylcyclohexane,
la 1,3,3-triméthyl-5,5-dipropylcyclohexylamine,
le 1-amino-1-méthyl-3(trans)-propylcyclohexane,
la 1-méthyl-3(cis)-propylcyclohexylamine,
le 1-amino-1-méthyl-3(trans)-éthylcyclohexane,
le 1-amino-1,3,3-triméthyl-5(cis)-éthylcyclohexane,
le 1-amino-1,3,3-triméthyl-5(trans)-éthylcyclohexane,
la cis-3-propyl-1,5,5-triméthylcyclohexylamine,
la trans-3-propyl-1,5,5-triméthylcyclohexylamine,
la N-éthyl-1,3,3,5,5-pentaméthylcyclohexylamine,
le N-méthyl-1-amino-1,3,3,5,5-pentaméthylcyclohexane,
le 1-amino-1-méthylcyclohexane,
le N,N-diméthyl-1-amino-1,3,3,5,5-pentaméthylcyclohexane,
la 2-(3,3,5,5-tétraméthylcyclohexyl)éthylamine,
la 2-méthyl-1-(3,3,5,5-tétraméthylcyclohexyl)propyl-2-amine,
le 2-(1,3,3,5,5-pentaméthylcyclohexyl)éthylamine semihydrate,
la N-(1,3,3,5,5-pentaméthylcyclohexyl)pyrrolidine,
le 1-amino-1,3(trans),5(trans)-triméthylcyclohexane,
le 1-amino-1,3(cis),5(cis)-triméthylcyclohexane,
le 1-amino-(1R,5S)trans-5-éthyl-1,3,3-triméthylcyclohexane,
le 1-amino-(1S,5S)cis-5-éthyl-1,3,3-triméthylcyclohexane,
le 1-amino-1,5,5-triméthyl-3(cis)-isopropylcyclohexane,
le 1-amino-1,5,5-triméthyl-3(trans)-isopropylcyclohexane,
le 1-amino-1-méthyl-3(cis)-éthylcyclohexane,
le 1-amino-1-méthyl-3(cis)-méthylcyclohexane,
le 1-amino-5,5-diéthyl-1,3,3-triméthylcyclohexane,
le 1-amino-1,3,3,5,5-pentaméthylcyclohexane,
le 1-amino-1,5,5-triméthyl-3,3-diéthylcyclohexane,
le 1-amino-1-éthyl-3,3,5,5-tétraméthylcyclohexane,
le N-éthyl-1-amino-1,3,3,5,5-pentaméthylcyclohexane,
la N-(1,3,5-triméthylcyclohexyl)pyrrolidine ou pipéridine,
la N-[1,3 (trans),5(trans)-triméthylcyclohexyl]pyrrolidine ou pipéridine, la N-[1,3(cis),5(cis)-triméthylcyclohexyl]pyrrolidine ou pipéridine,
la N-(1,3,3,5-tétraméthylcyclohexyl)pyrrolidine ou pipéridine,
la N-(1,3,3,5,5-pentaméthylcyclohexyl)pyrrolidine ou pipéridine,
la N-(1,3,5,5-tétraméthyl-3-éthylcyclohexyl)pyrrolidine ou pipéridine,
la N-(1,5,5-triméthyl-3,3-diéthylcyclohexyl)pyrrolidine ou pipéridine,
la N-(1,3,3-triméthyl-cis-5-éthylcyclohexyl)pyrrolidine ou pipéridine,
la N-[(1S,5S)cis-5-éthyl-1,3,3-triméthylcyclohexyl]pyrrolidine ou pipéridine,
la N-(1,3,3-triméthyl-trans-5-éthylcyclohexyl)pyrrolidine ou pipéridine,
la N-[(1R,5S)trans-5-éthyl,3,3-triméthylcyclohexyl]pyrrolidine ou pipéridine,
la N-(1-éthyl-3,3,5,5-tétraméthylyclohexyl)pyrrolidine ou pipéridine,
la N-(1-propyl-3,3,5,5-tétraméthylcyclohexyl)pyrrolidine ou pipéridine,
la N-(1,3,3,5,5-pentaméthylcyclohexyl)pyrrolidine,
et les isomères optiques, diastéréoisomères, énantiomères, hydrates, sels pharmaceutiquement acceptables et mélanges de ceux-ci.

10. Utilisation selon la revendication 1, dans laquelle le dérivé de 1-aminocyclohexane est choisi parmi le néramexane et les pro-médicaments, sels, isomères, analogues et dérivés de celui-ci.

11. Utilisation selon la revendication 10, dans laquelle le dérivé de 1-aminocyclohexane est le néramexane.

12. Utilisation selon l'une quelconque des revendications précédentes, dans laquelle le mammifère est un être humain.

13. Utilisation selon l'une quelconque des revendications précédentes, dans laquelle le médicament est préparé pour une administration du dérivé de 1-aminocyclohexane en une quantité thérapeutiquement efficace.

14. Utilisation selon la revendication 13, dans laquelle la quantité est comprise dans la plage allant de 1 à 100 mg/jour.

15. Utilisation selon la revendication 14, dans laquelle la quantité est comprise dans la plage allant de 5 à 60 mg/jour.

16. Utilisation selon la revendication 15, dans laquelle la quantité est comprise dans la plage allant de 10 à 40 mg/jour.

17. Utilisation selon l'une quelconque des revendications précédentes, dans laquelle le médicament comprend en outre un support ou un excipient pharmaceutiquement acceptable.

18. Utilisation selon l'une quelconque des revendications précédentes, dans laquelle le médicament est préparé pour une administration du dérivé de 1-aminocyclohexane de manière simultanée ou séquentielle avec un autre dérivé de 1-aminocyclohexane, un inhibiteur de l'acétylcholinestérase, un modificateur de la sécrétase ou une combinaison de ceux-ci.

19. Utilisation selon la revendication 18, dans laquelle l'inhibiteur de l'acétylcholinestérase est choisi parmi la galantamine, la tacrine, le donépézil et la rivastigmine.
